# FASCICULE DE BREVET EUROPEEN

(11) **EP 1 723 972 B1**
(45) Date de publication et mention de la délivrance du brevet: **24.01.2018**
(21) Numéro de dépôt: 06290796.9
(22) Date de dépôt: 16.05.2006
(51) Int. Cl.: C12N 5/22, A61K 49/00

(54) **Vectorisation de dsRNA par des particules cationiques et leur utilisation pour produire un modèle de peau.**
Vektorisierung von dsRNA mit kationischen Partikeln und ihre Verwendung für die Herstellung eines Hautmodells
Vectorisation of dsRNA by cationic particles and their use for the production of a skin model

(30) Priorité: 19.05.2005 FR 0551304
(43) Date de publication de la demande: 22.11.2006
(73) Titulaire: L'Oréal, 75008 Paris (FR)
(72) Inventeur: Collin-Djangone, Christine, 60110 Amblainville (FR); Simonnet, Jean-Thierry, 94240 Cachan (FR)
(74) Mandataire: Pillet, Anne-Helene

(56) Documents cités:
- WO-A-03/101376
- WO-A-2004/046354
- WO-A-2005/007196
- KASPAR, R. [REPRINT AUTHOR] ET AL: "Reporter gene delivery and potent inhibition of gene expression by modified and unmodified siRNAs in a mouse skin model ." JOURNAL OF INVESTIGATIVE DERMATOLOGY, (APR 2005) VOL. 124, NO. 4, SUPPL. S, PP. A81. MEETING INFO.: 66TH ANNUAL MEETING OF THE SOCIETY-FOR-INVESTIGATIVE- DERMATOLOGY. ST LOUIS, MO, USA. MAY 04 -07, 2005. SOC INVESTIGAT DERMATOL. CODEN: JIDEAE. ISSN:, avril 2005 (2005-04), XP008062688
- SCHMOOK F P ET AL: "Comparison of human skin or epidermis models with human and animal skin in in-vitro percutaneous absorption" INTERNATIONAL JOURNAL OF PHARMACEUTICS 14 MAR 2001 NETHERLANDS, vol. 215, no. 1-2, 14 mars 2001 (2001-03-14), pages 51-56, XP002390502 ISSN: 0378-5173
- YANO JUNICHI ET AL: "Antitumor activity of small interfering RNA/cationic liposome complex in mouse models of cancer." CLINICAL CANCER RESEARCH : AN OFFICIAL JOURNAL OF THE AMERICAN ASSOCIATION FOR CANCER RESEARCH. 15 NOV 2004, vol. 10, no. 22, 15 novembre 2004 (2004-11-15), pages 7721-7726, XP002375948 ISSN: 1078-0432
- SPAGNOU SEBASTIEN ET AL: "Lipidic carriers of siRNA: differences in the formulation, cellular uptake, and delivery with plasmid DNA." BIOCHEMISTRY. 26 OCT 2004, vol. 43, no. 42, 26 octobre 2004 (2004-10-26), pages 13348-13356, XP008044038 ISSN: 0006-2960
- MA ZHENG ET AL: "Cationic lipids enhance siRNA-mediated interferon response in mice." BIOCHEMICAL AND BIOPHYSICAL RESEARCH COMMUNICATIONS. 13 MAY 2005, vol. 330, no. 3, 13 mai 2005 (2005-05-13), pages 755-759, XP004833703 ISSN: 0006-291X

## Description

La présente description se rapporte à la vectorisation d'oligonucléotide d'ARN double brin par des particules cationiques choisies parmi des micelles de tensioactifs, des micelles de polymères blocs ou non, des liposomes et niosomes cationiques, des oléosomes cationiques, des nanoémulsions cationiques, ainsi que des particules organiques ou inorganiques cationiques et les nanocapsules, et à l'utilisation des compositions comprenant l'association d'au moins un dsRNA et d'au moins une particule cationique sur des modèles de peaux.

La présente invention se rapporte à un procédé de préparation d'un modèle de peau comprenant une étape d'administration dans le milieu de culture dudit modèle d'une composition comprenant au moins un oligonucléotide d'ARN double brin associé à au moins une particule cationique d'une taille inférieure ou égale à 1 µm, de potentiel zeta compris entre 10 et 80 mV choisie parmi les micelles de tensioactifs, les micelles de polymères blocs, les liposomes de tensioactifs non ionique et cationiques, les niosomes, les oléosomes, les particules de nanoémulsions, les nanocapsules, les particules organiques ou les particules inorganiques. L'utilisation de substances présentant une activité spécifique, telle qu'une activité biologique spécifique, dans le domaine cosmétique tel que le soin de la peau ou le soin capillaire, mais aussi dans les domaines dermatologique et pharmaceutique, est recherchée.
Depuis peu, l'utilisation de dsRNA et, plus particulièrement, de siRNA (de 12 à 40 nucléotides) permet d'obtenir une activité d'inhibition spécifique de la synthèse d'une protéine cible.
Le mécanisme moléculaire mis en jeu fait intervenir des fragments d'ARN double brin constitués de 12 à 40 nucléotides. La dégradation de l'ARNm cible est obtenue par l'activation du complexe RISC (RNA Induced Silencing Complex) effective par la fixation du brin anti-sens du dsRNA sur l'ARNm. Ces oligonucléotides d'ARN double brin, sont aussi appelés dsRNA ou encore siRNA (pour «short interfering» RNA), voir Tuschl T. Chem. Biochem. 2001 ;2 :239-245, Nykanen A & al Cell 2001 107 309-321, Dorsett Y. Nature, April 2004, Vol.3, page 318-329 et Downward J. BMJ 2004, 328, 1245-1248.
Cependant, le problème de la pénétration lors de l'application topique sur peau, muqueuses ou modèle de peau de ces siRNA, dont le poids moléculaire peut être d'environ 15-17 kD, se pose et demande à être optimisé. En effet, pour que l'activité spécifique relative au siRNA sélectionnée soit effective, il est nécessaire que celui-ci pénètre dans la cellule cible (par exemple les kératinocytes ou les mélanocytes...) jusque dans son cytoplasme. Or le *statum comeum,* siège de la fonction barrière cutanée, est difficile à pénétrer. Il reste donc à rechercher une solution permettant la pénétration de siRNA dans les modèles de peau.
De manière inattendue, l'association de siRNA avec des particules cationiques permet d'améliorer très significativement leur pénétration dans les cellules cibles d'un tissu tel que la peau, notamment des modèles de peau. Une fois pénétré, le siRNA devient actif. La pénétration peut être évaluée à l'aide de marqueur fluorescent fixé sur le siRNA et son activité par la quantification du messager ciblé par PCR quantitative ou par le dosage de la protéine correspondante au messager ciblé. Les particules cationiques de l'invention, peuvent être des micelles de tensioactifs, des micelles de polymères blocs ou non, des liposomes et niosomes cationiques, des oléosomes cationiques, des nanoémulsions cationiques, ainsi que des particules organiques ou inorganiques cationiques et les nanocapsules. D'une façon générale, la taille moyenne des particules de l'invention, doit être inférieure au µm.

La demande WO 03/101376 décrit des compositions cosmétiques comprenant au moins un oligonucléotide d'ARN double brin. Dans cette demande, l'oligonucléotide est nécessairement complexé avec un polymère cationique comme par exemple la PEI ou le chitosan, ce complexe oligonucléotide-polymère cationique pouvant ensuite être encapsulé dans des liposomes ou des niosomes et/ou adsorbé à la surface de particules telles que les liposomes, niosomes, oléosomes, nanosphères et nanocapsules.
Ainsi, dans ce document, le dsRNA est nécessairement complexé avec un polymère cationique avant d'être associé à un type de particule.
De plus, les micelles de tensioactifs ne sont pas décrites comme solution galénique performante pour la vectorisation de siRNA.

Dans l'article: *Intercellular adhesion molecule*-*1 suppression in skin by topical delivery of anti*-*sense oligonucteotides de* Mehta et al (J.Invest. Dermatol 115:805-812, 2000*),* les auteurs véhiculent de l'ADN simple brin dans une émulsion contenant 25% de tensioactifs (10% de glycéryl stearate et 15% de stéarate 40 OE). Outre le fait que cet article ne décrive pas les siRNA, la solution galénique proposée n'est pas compatible avec une bonne tolérance. En effet, les 25% de tensioactifs auront pour effet de détruire la fonction barrière de la peau, augmentant ainsi sa perméabilité aux éléments extérieurs et favorisant dramatiquement sa déshydratation : résultats qui ne sont pas recherchés dans les domaines d'application envisagés par la présente invention.

Dans la publication : *lipidic carriers of siRNA : differences in the formulation, cell uptake and delivery with plasmid DNA de* Spagnou et al (Biochemistry, 43:13348-56, 2004*),* les auteurs utilisent des liposomes cationiques tels que la lipofectamine 2000 ou des liposomes de DOPE « cationisés » avec du cholesteryloxycarbonyl 3-7 diazanonane 1-9 diamine (CDAN). Pour des raisons d'innocuité, la lipofectamine ne peut être utilisée sur peau humaine et on cherchera des solutions plus faciles à mettre en oeuvre et économiquement compatibles avec la mise sur le marché du produit.

D'autres publications (Yano Junichi et al Clinical Cancer Research, 2004, Spagnou et al. Biochemistry, 2004, et Ma Zheng et al. Biochemical and Biophysical Research Communication, 2004) ont proposé d'associer des siRAN à des vésicules cationiques constituées de lipides à chaînes oleyles comportant des insaturations les rendant très sensibles à l'oxydation et de phospholipides, ces vésicules sont destinées à être utilisées immédiatement après préparation car ne sont pas stables dans le temps.
La demande de brevet WO2004/046354 décrit l'utilisation de siRNA pour les dysfonctionnements cutanés et de préférence au niveau du derme. Les compositions décrites telles que les PIT, les émulsions W/O, O/W et W/O/W sont toutes non ioniques. Il n'est pas fait état dans cette demande de brevet de l'intérêt majeur et essentiel d'utiliser des tensioactifs cationiques dans des particules cationiques pour favoriser la pénétration des siRNA dans les structures cutanées. Par ailleurs, si les PIT peuvent être inférieures au µm, elles sont nécessairement non ioniques, comme décrit dans le brevet WO96/28132.
Le document WO 03/106636 décrit l'association d'un polynucléotide avec un tensioactif cationique tel que le Cétyl Tri Ammonium Chloride pour former un complexe. Il est nécessaire ensuite de stabiliser ce complexe par une étape d'incubation à une température comprise entre 35 et 50°C.
Dans un second temps, il est possible de déshydrater le mélange pour en obtenir une poudre qui sera ensuite diluée dans un solvant choisi, avant usage (injection). Il est également possible, une fois le complexe formé, de le stabiliser par l'ajout d'un lipide amphiphile qui peut être un tensioactif non ionique. Cette association ternaire est ensuite déshydratée pour obtenir une poudre qui sera ensuite diluée dans une solution aqueuse adaptée.
La demande WO 03/106636 ne précise pas qu'il faille se situer au dessus de la CMC (Concentration Micellaire Critique) du tensioactif cationique pour former le complexe polynucléotide/tensioactif cationique, le complexe pouvant se former en dessous de la CMC. Ce n'est qu'une fois ce complexe formé qu'il est envisagé l'ajout d'un autre composé amphiphile qui pourra former des micelles. On a ainsi l'intégration d'un complexe Polynucléotide/tensioactif cationique dans des micelles d'amphiphiles.
Ainsi dans ce document, il n'est pas question d'associer des particules cationiques à un polynucléotide.

La présente description concerne l'association de particules cationiques avec au moins un dsRNA qui va adhérer à la surface de la particule, celle-ci servant de véhicule pour le faire pénétrer dans les structures cutanées et dans les cellules cibles de la peau, en particulier des modèles de peau.

Ainsi un premier objet de la description se rapporte à un kit comprenant un modèle de peau et une composition comprenant au moins un oligonucléotide d'ARN double brin associé à au moins une particule cationique d'une taille inférieure ou égale à 1 µm, de potentiel zeta compris entre 10 et 80 mV choisie parmi les micelles dé tensioactifs, de préférence, les micelles de tensioactifs amphiphiles non ioniques et de tensioactifs cationiques, les micelles dé polymères blocs, de préférence, les micelles de polymère bloc amphiphile cationique, les micelles de polymère bloc amphiphile non ionique et de polymère bloc amphiphile cationique et les micelles de polymère bloc amphiphile non ionique et de tensioactif cationique, les liposomes de tensioactifs non ionique et cationiques, les niosomes, les oléosomes, les particules de nanoémulsions, les nanocapsules, les particules organiques ou les particules inorganiques.
La présente invention a pour objet un procédé de préparation d'un modèle de peau, peaux reconstruites ou peaux ex-vivo maintenues en survie, comprenant une étape d'administration dans le milieu de culture dudit modèle d'une composition comprenant au moins un oligonucléotide d'ARN double brin associé à au moins une particule cationique d'une taille inférieure ou égale à 1 µm, de potentiel zeta compris entre 10 et 80 mV choisie parmi les micelles de tensioactifs, les micelles de polymères blocs, les liposomes de tensioactifs non ionique et cationiques, les niosomes, les oléosomes, les particules de nanoémulsions, les nanocapsules, les particules organiques ou les particules inorganiques.
Les particules cationiques utilisées pour mettre en oeuvre l'invention sont des particules ayant une taille inférieure ou égale à 1 µm, de préférence inférieure ou égale à 500 nm, encore préférentiellement inférieure ou égale à 300 nm, mesurable avec, par exemple, un granulométre laser type B190 plus de la société Brookchaven, et ayant un potentiel zeta compris entre 10 et 80 mV pouvant être mesuré avec un zetamètre type DELSA 440 de la société Coultronics. Sont listées ci-après une liste non exhaustive des particules cationiques utilisables selon l'invention.

### Micelles de tensioactifs

Pour rappel, les micelles sont les agrégats que forment spontanément les molécules amphiphiles lorsqu'elles sont solubilisées dans l'eau ou l'huile, au-delà d'une certaine concentration dite critique : la CMC.
Les micelles utilisables dans le cadre de l'invention sont constituées d'au moins un tensioactif cationique. Ce tensioactif cationique peut être associé à un ou plusieurs tensioactifs amphiphiles non ioniques.
L'homme du métier sélectionnera avantageusement les tensioactifs non ioniques et cationiques dans le McCutcheons 1998 « emulsifiers and detergents » ainsi que dans les éditions suivantes. A titre d'exemples non limitatifs, les tensioactifs cationiques utilisables dans le cadre de l'invention sont listés ci-après.
Sans que ce soit limitatif, les tensioactifs non ioniques utilisables sont : les alkyls et polyalkyls (C6 à C30, saturé ou non, ramifié ou non) ester ou ether de POE, de glycérol et de polyglycérol, de sorbitan oxyéthyléné ou non, de sucrose, de glucose oxyethyléné ou non, de maltose, de POP-POE.

En cas de mélange entre tensioactifs non ioniques et tensioactifs cationiques, leurs proportions respectives, en poids, seront comprises entre 99/1 et 1/99.
Le taux de tensioactifs formant les micelles sera dépendant de la CMC de ceux-ci. Cependant, dans le cadre de l'invention, la concentration en tensioactifs micellaires sera comprise entre 0.1 et 10% et de préférence entre 0.2 et 5% en poids par rapport au poids total de la composition.

### Micelles de polymère bloc

Les micelles de polymères blocs amphiphiles peuvent être préparées selon le procédé décrit dans le document WO 04/035013.
Les copolymères blocs utiles pour la préparation des micelles associés aux dsRNA selon l'invention sont notamment des polymères blocs amphiphiles, préférentiellement non ioniques, diblocs ou triblocs, qui peuvent former au contact de l'eau des micelles. Ils sont notamment de type dibloc (A-B) ou tribloc (A-B-A), A correspondant à un bloc polymérique hydrophile non ionique et B à un bloc polymérique hydrophobe. Le poids moléculaire des polymères peut être compris entre 1000 et 100.000 et le rapport A/B peut être compris entre 1/100 et 50/1.
Le bloc polymérique hydrophile non ionique peut être choisi parmi le polyoxyde d'éthylène (POE) et la polyvinylpyrrolidone (PVP), le poly acide acrylique (PAA).
Le bloc polymérique hydrophobe peut être choisi parmi le polystyrène, le poly(tert.-butylstyrène), le poly(méthylméthacrylate), le poly(éthylacrylate), le poly(butylacrylate), le poly(butylméthacrylate), le poly(vinylacétate), les polycaprolactones, les polycaprolactames, les polydiméthylsiloxanes, les polyoxydes d'alkylène en C3-C6, le poly(acide aspartique), le poly(acide lactique), le poly(acide glycolique), la polyleucine, les polybutadiènes, les polyéthylènes, les polypropylènes, les polybutylènes.
Le copolymère bloc est choisi de préférence parmi les copolymères blocs suivants :
- polyoxyde de propylène/polyoxyde d'ethylène
- polystyrène/polyoxyéthylène
- polyméthylméthacrylate/polyoxyéthylène
- polybutylméthacrylate/polyoxyéthylène
- polyoxybutylène/polyoxyéthylène
- polycaprolactone/polyoxyéthylène
- polyéthylène/polyoxyéthylène
- polyoxyéthylène/polyoxybutylène/polyoxyéthylène.

Dans le cadre de l'invention, il est nécessaire d'ajouter à la composition micellaire :
- un polymère bloc amphiphile cationique dont l'un des blocs est cationique et peut être choisi parmi, à titre d'exemple :
   - Poly methacrylate de trimethyl ethyl ammonium ;
   - Polymethacrylate de dimethyl amino ethyl quaternisé ;
   - Poly methyl vinyl imidazolium ;
   - Poly vinyl benzyl trimethylamonium chlorure.
   l'association d'un polymère bloc amphiphile non ionique avec un polymère blocs amphiphile cationique est telle que le rapport entre les deux sera compris entre 99/1 et 1/99 ;
   et/ou
- au moins un tensioactif cationique tel que listé ci-après.
Dans ce cas, le rapport respectif entre le polymère bloc amphiphile non ionique et le tensioactif cationique sera compris entre 50/50 et 99/1.

Dans le cadre de l'invention, la concentration en polymères blocs micellaires associés ou non à un tensioactif cationique, sera comprise entre 0,1 et 10% et, de préférence, entre 0,2 et 5% en poids par rapport au poids total de la composition.

Dans une variante de l'invention, on peut également former des micelles constituées de polymères blocs amphiphile cationique tels que décrits ci-dessus.

### Liposomes et niosomes

Les lipides amphiphiles non ioniques aptes à former des liposomes non ioniques sont en particulier ceux décrits dans la demande de brevet EP 0 582 503.
En particulier, les lipides amphiphiles non-ioniques peuvent être constitués par un mélange d'esters d'au moins un polyol choisi dans le groupe formé par le polyéthylèneglycol comportant de 1 à 60 unités oxyde d'éthylène, le sorbitane, le sorbitane portant 2 à 60 unités d'oxyde d'éthylène, le glycérol portant 2 à 30 unités d'oxyde d'éthylène, les polyglycérols comportant 2 à 15 unités glycérol, les sucroses, les glucoses portant 2 à 30 unités oxyde d'éthylène, et d'au moins un acide gras comportant une chaîne alkyle en C5-C17, saturée ou non saturée, linéaire ou ramifiée, le nombre de chaînes alkyle par groupe polyol étant compris entre 1 et 10 .
L'expression "mélange d'esters" couvre non seulement des mélanges d'esters purs de familles chimiques différentes mais couvre également tout produit, qui contient plusieurs esters de polyol chimiquement purs de la même famille dans des proportions variables. C'est, en particulier, le cas des produits ayant une formule statistique, dans leur partie hydrophile, par exemple un ester de polyglycérol de formule CO-(OCH₂-CHOH-CH₂)ₙ-OH où n est une valeur statistique et qui peut contenir des proportions diverses d'esters pour lesquels n = 1, n = 2, n = 3, n = 4, etc ... ; c'est aussi le cas des esters comportant plusieurs chaînes alkyles dans leur partie lipophile, tels que les cocoates, qui contiennent des chaînes alkyles en C5 à C17 ou les isostéarates, où les chaînes alkyles en C17 sont un mélange complexe de formes isomères ; c'est également le cas des produits constitués par des mélanges de mono-, di-, tri-ou polyesters d'un même polyol. Il faut noter qu'un produit, qui ne contiendrait qu'un seul ester susceptible de former des vésicules et des impuretés d'un autre type ne pourrait pas être utilisé selon l'invention.
Des esters commerciaux utilisables seuls selon l'invention, car ils sont en réalité des mélanges d'esters, sont par exemple les suivants :
- les esters partiels de sorbitane (ou anhydride de sorbitol) et d'acide gras, vendus sous les dénominations commerciales "SPAN 20, 40, 60 et 80" par la société "ICI" ;
- l'isostéarate de sorbitane, vendu sous la dénomination commerciale "SI 10 R NIKKOL" par la société "NIKKO" ;
- le stéarate de sorbitane portant 4 unités oxyde d'éthylène, vendu sous la dénomination "TWEEN 61" par la société "ICI" ;
- le stéarate de polyéthylène glycol à 8 unités oxyde d'éthylène, vendu sous la dénomination "MYR J 45" par la société "ICI" ;
- le monostéarate du polyéthylène glycol de formule EMI6.1 formule dans laquelle n est égal à 4, vendu sous la dénomination "MYS 4" par la société "NIKKO" ;
- le stéarate de polyéthylène glycol de poids moléculaire 400, qualité chimique ou qualité produite par biotechnologie, vendu par la société "UNICHEMA" ;
- le stéarate de diglycéryle portant 4 unités d'oxyde d'éthylène, vendu sous la dénomination "HOSTACERINE DGS" par la société "HOECHST" ;
- le stéarate de tétraglycérol, vendu sous la dénomination 'TETRAGLYN 1S" par la société "NIKKO" ;
- l'isostéarate de diglycéryle, vendu par la société "SOLVAY" ;
- le distéarate de diglycéryle, vendu sous la dénomination "EMALEX DSG 2" par la société "NIHON" ;
- les mono-, di- et tripalmitostéarate de sucrose, vendus sous les dénominations " F50, F70, F110 ET F160 CRODESTA" par la société "CRODA" ;
   le mélange de mono- et dipalmitostéarate de sucrose, vendu sous la dénomination "GRILLOTEN PSE 141 G" par la société "GRILLO" ;
- le mélange de stéarate de sucrose et de cocoate de sucrose, vendu sous la dénomination "ARLATONE 2121" par la société "ICI" ;
- le distéarate de méthylglucose portant 20 unités oxyde d'éthylène, vendu sous la dénomination "GLUCAM E 20 DISTEARATE" par la société "AMERCHOL".

Des mélanges entre eux de ces différents produits, qui sont déjà des mélanges, ou des mélanges de ces produits avec des produits purs peuvent, bien entendu, être utilisés.
Les tensioactifs cationiques sont choisis dans la liste ci-après et de telle sorte qu'ils confèrent à la dispersion un pH compris entre 5 et 8 ; le rapport en poids entre la quantité de lipides amphiphiles non ioniques et celle de tensioactifs cationiques dans la phase lipidique étant compris entre 50/1 et 50/25 et le rapport en poids entre la phase lipidique et la phase aqueuse de dispersion étant compris entre 1/1 000 et 300/1 000.

### Oléosomes

Les oléosomes relatifs à l'invention sont décrits dans la demande de brevet EP 0 705 593. Il s'agit d'une émulsion du type huile dans eau formée de globules huileux pourvus d'un enrobage cristal liquide lamellaire dispersés dans une phase aqueuse, caractérisée en ce que chaque globule huileux est unitairement enrobé d'une couche monolamellaire ou oligolamellaire (1 à 10 feuillets visualisables en microscopie électronique à transmission après cryofracture) obtenue à partir d'au moins un agent tensioactif lipophile, d'au moins un agent tensioactif hydrophile et d'au moins un tensioactif cationique conférant à l'émulsion un pH allant de 5 à 8, les globules huileux enrobés ayant un diamètre moyen inférieur à 500 nanomètres.

L'agent tensioactif lipophile et l'agent tensioactif hydrophile comportent chacun au moins une chaîne grasse saturée ayant plus de 12 atomes de carbone environ. Plus préférentiellement encore, cette chaîne grasse contient de 16 à 22 atomes de carbone.
Selon un autre mode de réalisation préférentiel de l'invention, l'agent tensioactif lipophile présente un HLB compris entre environ 2 et environ 5. Comme cela est bien connu, on entend par HLB (Hydrophilic-Lipophilic Balance) l'équilibre entre la dimension et la force du groupe hydrophile et la dimension et la force de groupe lipophile de l'agent tensioactif.
Des exemples de tels agents tensioactifs lipophiles sont le distéarate de sucrose, le distéarate de diglycéryle, le tristéarate de tétraglycéryle, le décastéarate de décaglycéryle, le monostéarate de diglycéryle, le tristéarate d'hexaglycéryle, le pentastéarate de décaglycéryle, le monostéarate de sorbitane, le tristéarate de sorbitane, le monostéarate de diéthylène glycol, l'ester de glycérol et d'acides palmitique et stéarique, le monostéarate polyoxyéthylèné 2 OE (comportant 2 motifs oxyéthylène), le mono et dibéhénate de glycéryle, le tétrastéarate de pentaérythritol.
L'agent tensioactif hydrophile présente de préférence un HLB compris entre environ 8 et environ 12.
On peut citer comme exemples de tels tensioactifs hydrophiles les composés suivants : le monostéarate de sorbitane polyoxyéthyléné 4 OE, le tristéarate de sorbitane polyoxyéthyléné 20 OE, le monostéarate polyoxyéthyléné 8 OE, le monostéarate d'hexaglycéryle, le monostéarate polyoxyéthyléné 10 OE, le distéarate polyoxyéthylèné 12 OE et le distéarate de méthylglucose polyoxyéthyléné 20 OE.
Les tensioactifs cationiques peuvent être choisis parmi les composés cités ci-après

### Nanoémulsions

Les particules cationiques peuvent aussi être choisies parmi les nanoémulsions huile-dans-eau comportant une phase huileuse dispersée dans une phase aqueuse, dont les globules d'huile ont une taille moyenne en nombre inférieure à 100 nm, caractérisées en ce qu'elles comprennent au moins un lipide amphiphile comprenant au moins un lipide amphiphile non ionique et un lipide amphiphile cationique, la phase huileuse et les lipides amhiphiles étant présents en une teneur telle que le rapport pondéral phase huileuse/lipide amphiphile va de 3 à 10.
Les nanoémulsions ont généralement un aspect transparent à bleuté. Leur transparence se mesure par un coefficient de transmittance à 600 nm allant de 10 à 90 % ou bien par une turbidité. La turbidité des compositions de l'invention va de 60 à 400 NTU et de préférence de 70 à 300 NTU, turbidité mesurée au turbidimètre portatif HACH - Modèle 2100 P à environ 25°C.

Les globules d'huile des nanoémulsions de l'invention ont une taille moyenne en nombre, inférieure à 100 nm et de préférence allant de 20 à 80 nm et plus préférentiellement de 40 à 60 nm. La diminution de la taille des globules permet de favoriser la pénétration des actifs dans les couches superficielles de la peau (effet véhicule).

Les nanoémulsions conformes à l'invention sont préparées de préférence à des températures allant de 4 à 45°C et sont ainsi compatibles avec des actifs thermosensibles.

Ces dispersions sont notamment décrites dans les demandes suivantes : EP 0728 460, EP 0 879 589, EP 1 010 413, EP 1 010 414, EP 1 010 416, EP 1 013 338, EP 1 016 453, EP 1 018 363, EP 1 025 898 et EP 1 120 102. Dans toutes ces demandes, il est précisé que pour améliorer la stabilité des particules, on ajoutera un tensioactif ionique au tensioactif non ionique (ou mélange).

Dans le cas de la présente demande, on utilisera comme tensioactif ionique exclusivement un ou des tensioactifs cationiques.
Les proportions indiquées dans les références ci-dessus sont à conserver et à titre d'exemple de tensioactif cationique, on retiendra la liste commune à toutes les particules.

Le tensioactif non ioniques, de préférence hydrosolubles ou hydrodispersibles, comportent au moins une séquence hydrophobe et au moins une séquence hydrophile.
Les lipides amphiphiles non ioniques de l'invention sont préférentiellement choisis parmi :
1/ les tensioactifs siliconés,
2/ les lipides amphiphiles liquides à température inférieure ou égale à 45°C, choisis parmi les esters d'au moins un polyol d'au moins un acide gras comportant au moins une chaîne alkyle en C₈-C₂₂, saturée ou non saturée, linéaire ou ramifiée, et notamment insaturée ou ramifiée, le polyol étant choisi dans le groupe formé par le polyéthylèneglycol comportant de 1 à 60 unités d'oxyde d'éthylène, le sorbitan, le glycérol pouvant comporter de 2 à 30 unités d'oxyde d'éthylène, les polyglycérols comportant de 2 à 15 unités de glycérol,
3/ les esters d'acide gras et de sucre et les éthers d'alcool gras et de sucre,
4/ les tensioactifs solides à une température égale à 45°C choisis parmi les esters gras de glycérol, les esters gras de sorbitan et les esters gras de sorbitan oxyéthylénés, les éthers gras éthoxylés et les esters gras éthoxylés,
5/ Les copolymères blocs d'oxyde d'éthylène (A) et d'oxyde de propylène (B), et les mélanges de ces tensioactifs.

1/ Les tensioactifs siliconés utilisables selon l'invention sont des composés siliconés comportant au moins une chaîne oxyéthylénée -OCH₂CH₂- et/ou oxypropylénée - OCH₂CH₂CH₂-, on peut citer ceux décrits dans les documents US-A-5,364,633 et US-A-5,411,744.
   De préférence, le tensioactif siliconé utilisé selon la présente invention est un composé de formule (II) : 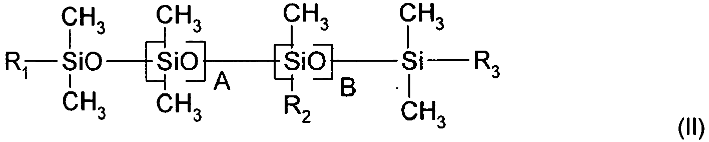 dans laquelle :
   R₁, R₂, R₃, indépendamment les uns des autres, représentent un radical alkyle en C₁-C₆ ou un radical -(CH₂)ₓ-(OCH₂CH₂)_{y}-(OCH₂CH₂CH₂)_{z}-OR₄, au moins un radical R₁, R₂ ou R₃ n'étant pas un radical alkyle ; R₄ étant un hydrogène, un radical alkyle ou un radical acyle ;
   A est un nombre entier allant de 0 à 200 ;
   B est un nombre entier allant de 0 à 50 ; à la condition que A et B ne soient pas égaux à zéro en même temps ;
   x est un nombre entier allant de 1 à 6 ;
   y est un nombre entier allant de 1 à 30 ;
   z est un nombre entier allant de 0 à 5.

   Selon un mode de réalisation préféré de l'invention, dans le composé de formule (X), le radical alkyle est un radical méthyle, x est un nombre entier allant de 2 à 6 et y est un nombre entier allant de 4 à 30.
   On peut citer, à titre d'exemple de tensioactifs siliconés de formule (II), les composés de formule (III) : 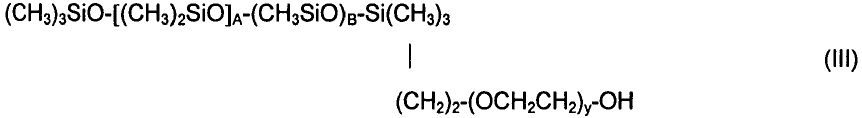 dans laquelle A est un nombre entier allant de 20 à 105, B est un nombre entier allant de 2 à 10 et y est un nombre entier allant de 10 à 20.
   On peut également citer à titre d'exemple de tensioactifs siliconés de formule (II), les composés de formule (IV) :

   H-(OCH₂CH₂)_{y}-(CH₂)₃-[(CH₃)₂SiO]_{A'}-(CH₂)₃-(OCH₂CH₂)_{y}-OH (IV)

   dans laquelle A' et y sont des nombres entiers allant de 10 à 20.
   On peut utiliser notamment comme tensioactifs siliconés, ceux commercialisés par la société Dow Corning sous les dénominations DC 5329, DC 7439-146, DC 2-5695 et Q4-3667. Les composés DC 5329, DC 7439-146, DC 2-5695 sont des composés de formule (XI) où respectivement A est 22, B est 2 et y est 12 ; A est 103, B est 10 et y est 12 ; A est 27, B est 3 et y est 12.
   Le composé Q4-3667 est un composé de formule (IV) où A est 15 et y est 13.
2/ Les lipides amphiphiles liquides à température inférieure ou égale à 45°C peuvent être notamment choisis parmi :
   - l'isostéarate de polyéthylèneglycol de poids molaire 400 (nom CTFA : PEG-8 Isostearate), vendu sous la dénomination Prisorine 3644 par la société UNICHEMA ;
   - l'isostéarate de diglycéryle, vendu par la société SOLVAY ;
   - le laurate de polyglycérol comportant 2 unités de glycérol (polyglyceryl-2 laurate), vendu sous la dénomination Diglycerin-monolaurate par la société SOLVAY ;
   - l'oléate de sorbitan, vendu sous la dénomination SPAN 80 par la société ICI ;
   - l'isostéarate de sorbitan, vendu sous la dénomination NIKKOL SI 10R par la société NIKKO ;
   - le cocoate d'α-butylglucoside ou le caprate d'α-butylglucoside commercialisés par la société ULICE.
3/ Les esters d'acide gras et de sucre, utilisables comme lipides amphiphiles non ioniques dans la nanoémulsion selon l'invention sont de préférence solides à une température inférieure ou égale à 45°C et peuvent être choisis notamment dans le groupe comprenant les esters ou les mélanges d'esters d'acide gras en C₈-C₂₂ et de sucrose, de maltose, de glucose ou de fructose, et les esters ou les mélanges d'esters d'acide gras en C₁₄-C₂₂ et de méthylglucose.
   Les acides gras en C₈-C₂₂ ou en C₁₄-C₂₂ formant le motif gras des esters utilisables dans la nanoémulsion de l'invention comportent une chaîne alkyle linéaire saturée ou non saturée, ayant respectivement de 8 à 22 ou de 14 à 22 atomes de carbone. Le motif gras des esters peut être notamment choisi parmi les stéarates, béhénates, arachidonates, palmitates, myristates, laurates, caprates et leurs mélanges. On utilise de préférence des stéarates.
   On peut citer, à titre d'exemple d'esters ou de mélanges d'esters d'acide gras et de sucrose, de maltose, de glucose ou de fructose, le monostéarate de sucrose, le distéarate de sucrose, le tristéarate de sucrose et leurs mélanges, tels que les produits commercialisés par la société Croda sous la dénomination Crodesta F50, F70, F110, F160 ayant respectivement un HLB (Hydrophilic Lipophilic Balance) de 5, 7, 11 et 16 ; et à titre d'exemple d'esters ou de mélanges d'esters d'acide gras et de méthylglucose, le distéarate de méthyl glucose et de polyglycérol-3, vendu par la société Goldschmidt sous la dénomination de Tego-care 450. On peut citer aussi les monoesters de glucose ou de maltose tels que l'o-hexadécanoyle-6-D-glucoside de méthyle et l'o-hexadécanoyle-6-D-maltoside.
   Les éthers d'alcool gras et de sucre, utilisables comme lipides amphiphiles non ioniques dans la nanoémulsion selon l'invention sont solides à une température inférieure ou égale à 45°C et peuvent être choisis notamment dans le groupe comprenant les éthers ou mélanges d'éthers d'alcool gras en C₈-C₂₂ et de glucose, de maltose, de sucrose ou de fructose et les éthers ou mélanges d'éthers d'alcool gras en C₁₄-C₂₂ et de méthylglucose. Ce sont notamment des alkylpolyglucosides.
   Les alcools gras en C₈-C₂₂ ou en C₁₄-C₂₂ formant le motif gras des éthers utilisables dans la nanoémulsion de l'invention comportent une chaîne alkyle linéaire saturée ou non saturée, ayant respectivement de 8 à 22 ou de 14 à 22 atomes de carbone. Le motif gras des éthers peut être notamment choisi parmi les motifs décyle, cétyle, béhényle, arachidyle, stéaryle, palmityle, myristyle, lauryle, capryle, hexadécanoyle, et leurs mélanges tels que cétéaryle.
   A titre d'exemple d'éthers d'alcool gras et de sucre, on peut citer les alkylpolyglucosides tels que le décylglucoside et le laurylglucoside commercialisés par exemple par la société Henkel sous les dénominations respectives de Plantaren 2000 et Plantaren 1200, le cétostéarylglucoside éventuellement en mélange avec l'alcool cétostéarylique, commercialisé par exemple sous la dénomination Montanov 68 par la société Seppic, sous la dénomination Tego-care CG90 par la société Goldschmidt et sous la dénomination Emulgade KE3302 par la société Henkel, ainsi que l'arachidylglucoside, par exemple sous la forme du mélange d'alcools arachidique et béhénique et d'arachidylglucoside, commercialisé sous la dénomination Montanov 202 par la société Seppic.
   On utilise plus particulièrement comme lipide amphiphile non ionique de ce type, le monostéarate de sucrose, le distéarate de sucrose, le tristéarate de sucrose et leurs mélanges, le distéarate de méthyl glucose et de polyglycérol-3 et les alkylpolyglucosides.
4/ Les esters gras de glycérol, utilisables comme lipides amphiphiles non ioniques dans la nanoémulsion selon l'invention, solides à une température inférieure ou égale à 45°C, peuvent être choisis notamment dans le groupe comprenant les esters formés d'au moins un acide comportant une chaîne alkyle linéaire saturée, ayant de 16 à 22 atomes de carbone, et de 1 à 10 motifs glycérol. On peut utiliser un ou plusieurs de ces esters gras de glycérol dans la nanoémulsion de l'invention.
   Ces esters peuvent être notamment choisis parmi les stéarates, béhénates, arachidates, palmitates et leurs mélanges. On utilise de préférence des stéarates et palmitates.
   On peut citer à titre d'exemple de tensioactif utilisable dans la nanoémulsion de l'invention, les monostéarate, distéarate, tristéarate et pentastéarate de décaglycérol (10 unités de glycérol) (noms CTFA : Polyglyceryl-10 stearate, Polyglyceryl-10 distearate, Polyglyceryl-10 tristearate, Polyglyceryl-10 pentastearate) tels que les produits vendus sous les dénominations respectives Nikkol Decaglyn 1-S, 2-S, 3-S et 5-S par la société Nikko, et le monostéarate de diglycérol (nom CTFA : Polyglyceryl-2 stearate) tel que le produit vendu par la société Nikko sous la dénomination Nikkol DGMS.
   Les esters gras de sorbitan, utilisables comme lipides amphiphiles non ioniques dans la nanoémulsion selon l'invention, solides à une température inférieure ou égale à 45°C, sont choisis notamment dans le groupe comprenant les esters d'acide gras en C₁₆-C₂₂ et de sorbitan et les esters d'acide gras en C₁₆-C₂₂ et de sorbitan oxyéthylénés. Ils sont formés d'au moins un acide gras comportant au moins une chaîne alkyle linéaire saturée, ayant respectivement de 16 à 22 atomes de carbone, et de sorbitol ou de sorbitol éthoxylé. Les esters oxyéthylénés comportent généralement de 1 à 100 unités d'oxyde d'éthylène et de préférence de 2 à 40 unités d'oxyde d'éthylène (OE).
   Ces esters peuvent être notamment choisis parmi les stéarates, béhénates, arachidates, palmitates, et leurs mélanges. On utilise de préférence des stéarates et palmitates.
   On peut citer à titre d'exemple d'ester gras de sorbitan et d'ester gras de sorbitan oxyéthyléné, utilisable dans la nanoémulsion de l'invention, le monostéarate de sorbitan (nom CTFA : Sorbitan stearate) vendu par la société ICI sous la dénomination Span 60, le monopalmitate de sorbitan (nom CTFA : Sorbitan palmitate) vendu par la société ICI sous la dénomination Span 40, le tristéarate de sorbitan 20 OE (nom CTFA : Polysorbate 65) vendu par la société ICI sous la dénomination Tween 65.
   Les éthers gras éthoxylés solides à une température inférieure ou égale à 45°C, utilisables comme lipides amphiphiles non ioniques dans la nanoémulsion selon l'invention sont de préférence des éthers formés de 1 à 100 unités d'oxyde d'éthylène et d'au moins une chaîne d'alcool gras ayant de 16 à 22 atomes de carbone. La chaîne grasse des éthers peut être notamment choisie parmi les motifs béhényle, arachidyle, stéaryle, cétyle, et leurs mélanges tels que cétéaryle. A titre d'exemple d'éthers gras éthoxylés, on peut citer les éthers d'alcool béhénique comprenant 5, 10, 20 et 30 unités d'oxyde d'éthylène (noms CTFA : Beheneth-5, Beheneth-10, Beheneth-20, Beheneth-30), tels que les produits commercialisés sous les dénominations Nikkol BB5, BB10, BB20, BB30 par la société Nikko, et l'éther d'alcool stéarylique comprenant 2 unités d'oxyde d'éthylène (nom CTFA : Steareth-2), tel que le produit commercialisé sous la dénomination Brij 72 par la société ICI.
   Les esters gras éthoxylés solides à une température inférieure ou égale à 45°C, utilisables comme lipides amphiphiles non ioniques dans la nanoémulsion selon l'invention sont des esters formés de 1 à 100 unités d'oxyde d'éthylène et d'au moins une chaîne d'acide gras comportant de 16 à 22 atomes de carbone. La chaîne grasse des esters peut être notamment choisie parmi les motifs stéarate, béhénate, arachidate, palmitate, et leurs mélanges. A titre d'exemple d'esters gras éthoxylés, on peut citer l'ester d'acide stéarique comprenant 40 unités d'oxyde d'éthylène, tel que le produit commercialisé sous la dénomination Myrj 52 (nom CTFA : PEG-40 stearate) par la société ICI ainsi que l'ester d'acide béhénique comprenant 8 unités d'oxyde d'éthylène (nom CTFA : PEG-8 behenate), tel que le produit commercialisé sous la dénomination Compritol HD5 ATO par la société Gattefosse.
5/ Les copolymères blocs d'oxyde d'éthylène et d'oxyde de propylène, utilisables comme lipides amphiphiles non ioniques dans la nanoémulsion selon l'invention peuvent être choisis notamment parmi les copolymères blocs de formule (V) :

   HO(C₂H₄O)x(C₃H₆O)y(C₂H₄O)zH (V)

   dans laquelle x, y et z sont des nombres entiers tels que x+z va de 2 à 100 et y va de 14 à 60, et leurs mélanges, et plus particulièrement parmi les copolymères blocs de formule (V) ayant un HLB allant de 2 à 16.

Ces copolymères blocs peuvent être notamment choisis parmi les poloxamers et notamment parmi le Poloxamer 231 tel que le produit commercialisé par la société ICI sous la dénomination Pluronic L81 de formule (V) avec x=z=6, y=39 (HLB 2) ; le Poloxamer 282 tel que le produit commercialisé par la société ICI sous la dénomination Pluronic L92 de formule (V) avec x=z=10, y=47 (HLB 6) ; et le Poloxamer 124 tel que le produit commercialisé par la société ICI sous la dénomination Pluronic L44 de formule (V) avec x=z=11, y=21 (HLB 16).

Comme lipides amphiphiles non ioniques, on peut aussi citer les mélanges de tensioactifs non ioniques décrits dans le document EP-A-705593 incorporé ici pour référence.

Parmi les lipides amphiphiles non ioniques, on peut utiliser en particulier :
- l'isostéarate de PEG 400 ou PEG-8 Isostéarate (comportant 8 moles d'oxyde d'éthylène),
- l'isostéarate de diglycéryle,
- le monolaurate de polyglycérol comportant 2 unités de glycérol et les stéarates de polyglycérol comportant 10 unités de glycérol,
- l'oléate de sorbitan,
- l'isostéarate de sorbitan,et leurs mélanges.

Les lipides amphiphiles non ionique peuvent être présents dans la nanoémulsion selon l'invention en une teneur allant de **0,2 % à 12 %** en poids, par rapport au poids total de la composition, et de préférence allant de **0,2 % à 8 %** en poids, et préférentiellement allant de **0,2 % à 6 %** en poids.
Les lipides amphiphiles cationiques sont choisis parmi la liste donnée ci après.
Ils sont présents dans les nanoémulsions de l'invention, de préférence, dans des concentrations allant de **0,01 à 6 %** en poids par rapport au poids total de la nanoémulsion et plus particulièrement de **0,2 à 4 %** en poids.

### Huiles :

La phase huileuse de la nanoémulsion selon l'invention comprend au moins une huile. Les huiles pouvant être utilisées dans les nanoémulsions de l'invention sont choisies préférentiellement dans le groupe formé par :
- les huiles d'origine animale ou végétale, formées par des esters d'acides gras et de polyols, en particulier les triglycérides liquides, par exemple les huiles de tournesol, de maïs, de soja, d'avocat, de jojoba, de courge, de pépins de raisin, de sésame, de noisette, les huiles de poisson, le tricaprocaprylate de glycérol, ou les huiles végétales ou animales de formule R₉COOR₁₀ dans laquelle R₉ représente le reste d'un acide gras supérieur comportant de 7 à 29 atomes de carbone et R₁₀ représente une chaîne hydrocarbonée linéaire ou ramifiée contenant de 3 à 30 atomes de carbone, en particulier alkyle ou alkényle, par exemple, l'huile de Purcellin ou la cire liquide de jojoba ;
- des huiles essentielles naturelles ou synthétiques telles que, par exemple, les huiles d'eucalyptus, de lavandin, de lavande, de vétivier, de litsea cubeba, de citron, de santal, de romarin, de camomille, de sarriette, de noix de muscade, de cannelle, d'hysope, de carvi, d'orange, de géraniol, de cade et de bergamote;
- les huiles de synthèse telles que l'huile de parléam, les polyoléfines et les esters d'acides carboxyliques liquides ;
- les huiles minérales telles que l'hexadécane, l'isohexadécane et l'huile de paraffine ;
- les huiles halogénés, notamment des fluorocarbures tels que des fluoramines par exemple la perfluorotributylamine, des hydrocarbures fluorés, par exemple le perfluorodécahydronaphtalène, des fluoroesters et des fluoroéthers ;
- les huiles de silicone volatiles ou non volatiles.

Les polyoléfines utilisables comme huiles de synthèse sont en particulier les poly-α-oléfines et plus particulièrement celles de type polybutène, hydrogéné ou non, et de préférence polyisobutène, hydrogéné ou non.
Les esters d'acides carboxyliques liquides utilisables comme huiles de synthèse, peuvent être des esters d'acides mono-, di-, tri- ou tétra-carboxyliques. Le nombre total de carbone des esters est généralement supérieur ou égal à 10 et de préférence inférieur à 100 et plus particulièrement inférieur à 80. Ce sont notamment les monoesters d'acides aliphatiques saturés ou insaturés, linéaires ou ramifiés en C₁-C₂₆ et d'alcools aliphatiques saturés ou insaturés, linéaires ou ramifiés en C₁-C₂₆, le nombre total de carbone des esters étant généralement supérieur ou égal à 10. On peut également utiliser les esters d'acides di- ou tri-carboxyliques en C₄-C₂₂ et d'alcools en C₁-C₂₂ et les esters d'acides mono-, di- ou tri-carboxyliques et d'alcools di-, tri-, tétra- ou penta-hydroxylés en C₂-C₂₆.
Parmi les esters cités ci-dessus, on préfère utiliser les palmitates d'alkyle tels que le palmitate d'éthyle, le palmitate d'isopropyle, le palmitate d'éthyl-2-héxyle, le palmitate de 2-octyldécyle ; les myristates d'alkyle tels que le myristate d'isopropyle, le myristate de butyle, le myristate de cétyle, le myristate de 2-octyldodécyle ; les stéarates d'alkyle tel que le stéarate d'hexyle, le stéarate de butyle, le stéarate d'isobutyle ; les malates d'alkyle tels que le malate de dioctyle ; les laurates d'alkyle tels que le laurate d'hexyle et le laurate de 2-hexyldécyle ; l'isononanate d'isononyle ; l'octanoate de cétyle.
Avantageusement, la nanoémulsion selon l'invention contient au moins une huile de poids moléculaire supérieur ou égal à 400, notamment allant de 400 à 10 000, mieux allant de 400 à 5000, ou encore allant de 400 à 5000. Les huiles de poids moléculaire supérieur ou égal à 400 peuvent être choisies parmi les huiles d'origine animale ou végétale, les huiles minérales, les huiles de synthèse et les huiles de silicone, et leurs mélanges. Comme huiles de ce type, on peut citer par exemple le palmitate d'isocétyle, le stéarate d'isocétyle, l'huile d'avocat, l'huile de jojoba.

Les nanoémulsions utilisées pour mettre en oeuvre l'invention comportent une quantité de phase huileuse (huile et autres corps gras hormis le lipide amphiphile) allant de préférence, de 2 à 40 % en poids par rapport au poids total de la nanoémulsion et plus particulièrement de 4 à 30 % en poids et préférentiellement de 4 à 20 % en poids.

La phase huileuse et les lipides amphiphiles (amphiphiles non ioniques et ioniques) sont de préférence présents dans la nanoémulsion selon l'invention selon un rapport pondéral de la quantité de phase huileuse sur la quantité de lipide amphiphile allant de 3 à 10 et préférentiellement allant de 3 à 6. On entend ici par "quantité de phase huileuse" la quantité totale des constituants de cette phase huileuse sans inclure la quantité de lipide amphiphile. Les nanoémulsions conformes à la présente invention peuvent contenir, en complément des dérivés d'urée de formule (I) décrits précédemment, des solvants, notamment pour améliorer, si nécessaire, la transparence de la composition.

Ces solvants sont choisis de préférence dans le groupe formé par :
- les alcools inférieurs en C₁-C₈ tels que l'éthanol ;
- les glycols tels que la glycérine, le propylèneglycol, le 1,3- butylèneglycol, le dipropylèneglycol, les polyéthylèneglycols comportant de 4 à 16 unités d'oxyde d'éthylène et de préférence de 8 à 12.
- les sucres tels que le glucose, le fructose, le maltose, le lactose, le sucrose.

Ces solvants peuvent être utilisés en mélange. Lorsqu'ils sont présents dans la nanoémulsion de l'invention, ils peuvent être utilisés à des concentrations allant de préférence de 0,01 à 30 % en poids par rapport au poids total de la nanoémulsion, et mieux de 5 à 20 % en poids par rapport au poids total de la nanoémulsion. La quantité d'alcool(s) et/ou de sucre(s) va de préférence de 5 à 20 % en poids par rapport au poids total de la nanoémulsion et la quantité de glycol(s) va de préférence de 5 à 15 % en poids par rapport au poids total de la nanoémulsion.

### Procédé de préparation :

Le procédé de préparation d'une nanoémulsion telle que définie ci-dessus consiste à mélanger la phase aqueuse contenant le dérivé d'urée et la phase huileuse, sous agitation vive, à une température allant de 10 °C à 80 °C, à effectuer une étape d'homogénéisation haute pression à une pression supérieure à 5.10⁷ Pa et à ajouter éventuellement le polymère utilisé. Selon un mode préféré de réalisation de l'invention, on effectue ensuite encore une étape d'homogénéisation haute pression à une pression supérieure à 5.10⁷ Pa. L'homogénéisation haute pression est réalisée de préférence à une pression allant de 6.10⁷ à 18.10⁷ Pa. Le cisaillement va de préférence de 2.10⁶ s⁻¹ à 5.10⁸ s⁻¹ et mieux de 1.10⁸ s⁻¹ à 3.10⁸ s⁻¹ (s⁻¹ signifie seconde⁻¹). Un tel procédé permet de réaliser des nanoémulsions compatibles avec des composés actifs thermosensibles, et pouvant contenir des huiles et notamment des parfums qui renferment des corps gras, sans les dénaturer.

### Nanocapsules

Les nanocapsules utilisées pour mettre en oeuvre l'invention sont celles décrites dans les demandes de brevet EP 0 447 318, EP 0 557 489, EP 0 780 115, EP 1 025 901, EP 1 029 587, EP 1 034 839, EP 1 414 390, FR 2 830 776, EP 1 342 471, FR 2 848 879 et FR 04/50057.
Les nanocapsules sont des particules Core-Shell ayant un coeur huileux et une coque polymérique. Les différentes demandes citées plus haut portent sur différentes familles de polymères et différents procédés pour les obtenir. La taille des capsules est toujours inférieure à 1 µm et il est possible d'avoir des tailles inférieures à 80 nm. Ces particules peuvent être enrobées par une phase cristal liquide lamellaire le plus souvent constituée d'une lécithine ou d'un diméthicone copolyol. L'enrobage devant être constitué d'un lipide amphiphile apte à former spontanément une phase cristal liquide lamellaire au contact de l'eau. C'est à ce lipide amphiphile apte à former une phase lamellaire que sera ajouté le tensioactif cationique qui confèrera à la particules (la nanocapsule) un potentiel zeta positif. Le rapport pondéral entre le lipide amphiphile formant la phase lamellaire et le tensioactif cationique sera compris entre 99/1 et 75/25.
Les tensioactifs cationiques utilisables sont ceux listés ci-après.

### Particules organiques

Les particules organiques utilisées pour mettre en oeuvre l'invention sont des nanosphères, pleines, sans cavité interne formée par différents procédés (dispersion dans l'eau, nanoprécipitation, microémulsion...) et composées d'au moins un polymère ou d'au moins un copolymère ou d'un mélange de ceux ci. La particule est cationique, avec le potentiel zeta défini précédemment, soit parce que le ou les polymères ou copolymères sont cationiques, soit parce que non ioniques et on utilise un tensioactif cationique tel que décrit ci-après. Par rapport au polymère, le taux de tensioactif cationique sera compris entre 0 et 25%.

### Particules inorganiques

Les particules inorganiques cationiques utilisées pour mettre en oeuvre l'invention peuvent être, à titre d'exemple, à base de silice, TiO2, ZnO, d'alumine... A titre d'exemple, on citera les particules d'alumine en dispersion colloïdale dans l'eau comme les Nanomer 2 de Nalco. Clariant et Grace, proposent également des particules de ce type.

### Tensioactifs cationiques utilisables pour la préparation des particules cationiques de l'invention

Les tensioactifs cationiques qui peuvent être utilisés pour l'invention sont listés ci-après, cette liste est non limitative.

Les lipides amphiphiles cationiques sont choisis, de préférence, dans le groupe formé par les sels d'ammonium quaternaire, les amines grasses et leurs sels.

Les sels d'ammonium quaternaires sont par exemple :
- ceux qui présentent la formule générale (IV) suivante : 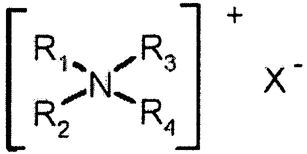 dans laquelle les radicaux R1 à R4, qui peuvent être identiques ou différents, représentent un radical aliphatique, linéaire ou ramifié, comportant de 1 à 30 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, polyoxyalkylène(C2-C6), alkylamide, alkyl(C12-C22)amido alkyle(C2-C6), alkyl(C12-C22)acétate, hydroxyalkyle, comportant environ de 1 à 30 atomes de carbone; X est un anion choisi dans le groupe des halogénures, phosphates, acétates, lactates, alkyl(C2-C6)sulfates, alkyl-ou-alkylarylsulfonates,
- les sels d'ammonium quaternaire de l'imidazolinium, comme par exemple celui de formule (V) suivante : 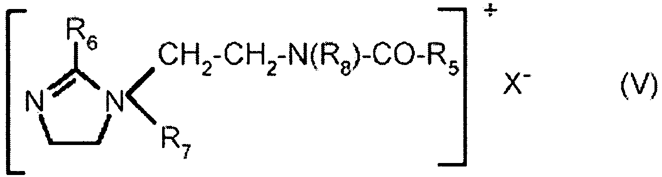 dans laquelle R5 représente un radical alcényle ou alkyle comportant de 8 à 30 atomes de carbone par exemple dérivés des acides gras du suif, R6 représente un atome d'hydrogène, un radical alkyle en C1-C4 ou un radical alcényle ou alkyle comportant de 8 à 30 atomes de carbone, R7 représente un radical alkyle en C1- C4 , R8 représente un atome d'hydrogène, un radical alkyle en C1-C4, X est un anion choisi dans le groupe des halogénures, phosphates, acétates, lactates, alkylsulfates, alkyl-ou-alkylarylsulfonates. De préférence, R5 et R6 désignent un mélange de radicaux alcényle ou alkyle comportant de 12 à 21 atomes de carbone par exemple dérivés des acides gras du suif, R7 désigne méthyle, R8 désigne hydrogène. Un tel produit est par exemple commercialisé sous la dénomination «REWOQUAT W 75» par la société REWO,
- les sels de diammonium quaternaire de formule (VI) : 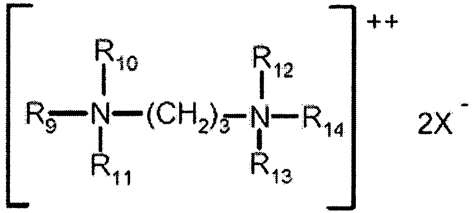 dans laquelle R9 désigne un radical aliphatique comportant environ de 16 à 30 atomes de carbone, R10, R11, R12, R13 et R14 , identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates. De tels sels de diammonium quaternaire comprennent notamment le dichlorure de propanesuif diammonium ;
- les sels d'ammonium quaternaire contenant au moins une fonction ester Les sels d'ammonium quaternaire contenant au moins une fonction ester utilisables selon l'invention sont par exemple ceux de formule (VII) suivante : 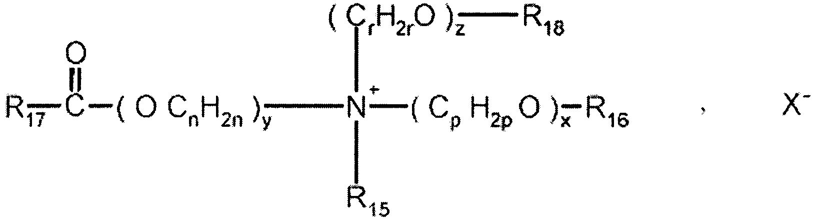 dans laquelle :
   - R15 est choisi parmi les radicaux alkyles en C1-C6 et les radicaux hydroxyalkyles ou dihydroxyalkyles en C1-C6 ;
   - R16 est choisi parmi :
   - le radical 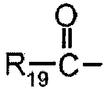
   - les radicaux R20 hydrocarbonés en C1-C22 linéaires ou ramifiés, saturés ou insaturés,
   - l'atome d'hydrogène,
   - R18 est choisi parmi :
   - le radical 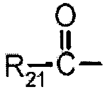
   - les radicaux R22 hydrocarbonés en C1-C6 linéaires ou ramifiés, saturés ou insaturés,
   - l'atome d'hydrogène,
   - R17, R19 et R21, identiques ou différents, sont choisis parmi les radicaux hydrocarbonés en C7-C21, linéaires ou ramifiés, saturés ou insaturés ;
   - n, p et r, identiques ou différents, sont des entiers valant de 2 à 6 ;
   - y est un entier valant de 1 à 10 ;
   - x et z, identiques ou différents, sont des entiers valant de 0 à 10 ;
   - X- est un anion simple ou complexe, organique ou inorganique ; sous réserve que la somme x + y + z vaut de 1 à 15, que lorsque x vaut 0 alors R16 désigne R20 et que lorsque z vaut 0 alors R18 désigne R22.
Les radicaux alkyles R15 peuvent être linéaires ou ramifiés et plus particulièrement linéaires.
De préférence R15 désigne un radical méthyle, éthyle, hydroxyéthyle ou dihydroxypropyle et plus particulièrement un radical méthyle ou éthyle. Avantageusement, la somme x + y + z vaut de 1 à 10.
Lorsque R16 est un radical R20 hydrocarboné, il peut être long et avoir de 12 à 22 atomes de carbone ou court et avoir de 1 à 3 atomes de carbone.
Lorsque R18 est un radical R22 hydrocarboné, il a de préférence 1 à 3 atomes de carbone. Avantageusement, R17, R19 et R21, identiques ou différents, sont choisis parmi les radicaux hydrocarbonés en C11-C21, linéaires ou ramifiés, saturés ou insaturés, et plus particulièrement parmi les radicaux alkyle et alcényle en C11-C21, linéaires ou ramifiés, saturés ou insaturés.
De préférence, x et z, identiques ou différents, valent 0 ou 1.
Avantageusement, y est égal à 1.
De préférence, n, p et r, identiques ou différents, valent 2 ou 3 et encore plus particulièrement sont égaux à 2.

L'anion est de préférence un halogénure (chlorure, bromure ou iodure) ou un alkylsulfate plus particulièrement méthylsulfate. On peut cependant utiliser le méthanesulfonate, le phosphate, le nitrate, le tosylate, un anion dérivé d'acide organique tel que l'acétate ou le lactate ou tout autre anion compatible avec l'ammonium à fonction ester.
L'anion X- est encore plus particulièrement le chlorure ou le méthylsulfate.

On utilise plus particulièrement les sels d'ammonium de formule (VII) dans laquelle :
- R15 désigne un radical méthyle ou éthyle,
- x et y sont égaux à 1 ;
- z est égal à 0 ou 1 ;
- n, p et r sont égaux à 2 ;
- R16 est choisi parmi :
   - le radical 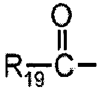
   - les radicaux méthyle, éthyle ou hydrocarbonés en C14-C22
   - l'atome d'hydrogène ;
   - R18 est choisi parmi :
   - le radical 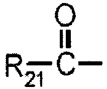
   - l'atome d'hydrogène ;
      R17, R19 et R21, identiques ou différents, sont choisis parmi les radicaux hydrocarbonés en C13-C17, linéaires ou ramifiés, saturés ou insaturés et de préférence parmi les radicaux alkyles et alcényle en C13-C17, linéaires ou ramifiés, saturés ou insaturés. Avantageusement, les radicaux hydrocarbonés sont linéaires.

On peut citer par exemple les composés de formule (VII) tels que les sels (chlorure ou méthylsulfate notamment) de diacyloxyéthyl diméthyl ammonium, de diacyloxyéthyl hydroxyéthyl méthyl ammonium, de monoacyloxyéthyl dihydroxyéthyl méthyl ammonium, de triacyloxyéthyl méthyl ammonium, de monoacyloxyéthyl hydroxyéthyl diméthyl ammonium et leurs mélanges. Les radicaux acyles ont de préférence 14 à 18 atomes de carbone et proviennent plus particulièrement d'une huile végétale comme l'huile de palme ou de tournesol.

Lorsque le composé contient plusieurs radicaux acyles, ces derniers peuvent être identiques ou différents.
Ces produits sont obtenus par exemple par estérification directe de la triéthanolamine, de la triisopropanolamine, d'alkyldiéthanolamine ou d'alkyldiisopropanolamine éventuellement oxyalkylénées sur des acides gras ou sur des mélanges d'acides gras d'origine végétale ou animale ou par transestérification de leurs esters méthyliques. Cette estérification est suivie d'une quaternisation à l'aide d'un agent alkylant tel qu'un halogénure d'alkyle (méthyle ou éthyle de préférence), un sulfate de dialkyle (méthyle ou éthyle de préférence), le méthanesulfonate de méthyle, le paratoluènesulfonate de méthyle, la chlorhydrine du glycol ou du glycérol.
De tels composés sont par exemple commercialisés sous les dénominations DEHYQUART par la société HENKEL, STEPANQUAT par la société STEPAN, NOXAMIUM par la société CECA, REWOQUAT WE 18 par la société REWOWITCO.

La composition utilisée pour mettre en oeuvre l'invention contient de préférence un mélange de sels de mono, di et triester d'ammonium quaternaire avec une majorité en poids de sels de diester.
Comme mélange de sels d'ammonium, on peut utiliser par exemple le mélange contenant 15 à 30 % en poids de méthylsulfate d'acyloxyéthyl dihydroxyéthyl méthyl ammonium, 45 à 60 % de méthylsulfate de diacyloxyéthyl hydroxyéthyl méthyl ammonium et 15 à 30 % de méthylsulfate de triacyloxyéthyl méthyl ammonium, les radicaux acyles ayant de 14 à 18 atomes de carbone et provenant d'huile de palme éventuellement partiellement hydrogénée. On peut aussi utiliser les sels d'ammonium contenant au moins une fonction ester décrits dans les brevets US-A-4874554 et US-A-4137180.
Parmi les sels d'ammonium quaternaire de formule (IV) on préfère, d'une part, les chlorures de tétraalkylammonium comme par exemple les chlorures de dialkyldiméthylammonium ou d'alkyltriméthylammonium, dans lesquels le radical alkyl comporte environ de 12 à 22 atomes de carbone, en particulier les chlorures de béhényltriméthylammonium, de distéaryldiméthylammonium, de cétyltriméthylammonium, de benzyl diméthyl stéaryl ammonium ou encore, d'autre part, le chlorure de stéaramidopropyldiméthyl (myristyl acétate) ammonium commercialisé sous la dénomination «CERAPHYL 70» par la société VAN DYK.
Selon l'invention, le chlorure ou le bromure de béhényltriméthylammonium et le CTAB (cétyltrimethylammonium bromine) sont les sels d'ammonium quaternaires les plus particulièrement préférés.

Les amines grasses de l'invention correspondent à la formule générale : 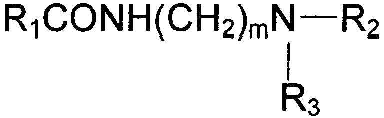
- R₁ est une chaîne hydrocarbonée saturée ou non, ramifiée ou non, ayant entre 8 et 30 atomes de carbone et de préférence entre 10 et 24.
- R₂ et R₃ sont sélectionnés indépendamment parmi hydrocarbonées saturées ou non, ramifiées ou non, ayant entre 1 et 10 atomes de carbone et de préférence entre 1 et 4.
- R₂ et R₃ peuvent également, toujours indépendamment l'un de l'autre correspondre à un atome d'hydrogène H .
- M est compris entre 1 et 10 et de préférence entre 1 et 5.
A titre d'exemple non limitatif, on citera : la stearylamine, le Stearate aminoethylethanolamide, Stearyl diethanolamide, Stearate diethylenetriamine, la stearamidopropyldimethylamine, la stearamidopropyldiethylamine, la stearamidoethyldiethylamine, la stearamidoethyldimethylamine, la palmitamidopropyldimethylamine, la palmitamidopropyldiethylamine, la palmitamidoethyldiethylamine, la palmitamidoethyldimethylamine, la behenamidopropyldimethylamine, la behenamidopropyldiethylmine, la behenamidoethyldiethylamine, la behenamidoethyldimethylamine, la arachidamidopropyldimethylamine, l'arachidamidopropyldiethylamine, l'arachidamidoethyldiethylamine, l'arachidamidoethyldimethylamine.
On peut citer comme amine grasse commercialement disponible, l'Incromine BB de Croda, l'Amidoamine MSP de Nikkol, les Lexamine de Inolex.
Comme autres amines grasses, on citera, à titre d'exemple, la stearylamine, le Stearate aminoethylethanolamide, la Stearyl diethanolamide, le Stearate diethylenetriamine que, entre autres, Sabo commecrcialise avec la série des Sabomina.
On citera également les acétates amines grasses comme la série des Acetamine de Kao Corp.
Ces amines grasses peuvent être également ethoxylées comme les Berol 380, 390, 453 et 455, les Ethomeen de Akzo Nobel ou les Marlazin L10, OL2, OL20, T15/2, T50 de Condea Chemie.

Les particules pour mettre en oeuvre la présente invention peuvent être introduites dans tous supports galéniques à visée cosmétique, dermatologique et ophtalmique. A titre d'exemple, on citera les lotions, les serums, les gels et tous les type d'émulsions.

Les oligonucléotides d'ARN double brin (ou encore dsRNA pour double stranded RNA, siRNA pour short interfering RNA) utilisables pour mettre en oeuvre la présente invention sont des fragments d'acides nucléiques doubles brins capables d'inhiber totalement ou partiellement l'expression d'un gène dans une cellule eucaryote, ces oligonucléotides d'ARN double brin comportent généralement entre 12 et 40 nucléotides, préférentiellement de 20 à 25 nucléotides. Ces oligonucléotides double brin sont composés d'un brin sens et d'un brin anti-sens correspondant à la séquence de l'ARN messager cible à dégrader. L'oligonucléotide d'ARN double brin présente des extrémités franches ou non appariées de 2 à 6 nucléotides.
Les oligonucléotides d'ARN double brin utilisées pour mettre en oeuvre la présente invention pourront être des oligonucléotides d'ARN double brin comportant un ou plusieurs nucléotides modifiés par substitution, délétion ou insertion, ces modifications seront telles que la séquence l'oligonucléotide d'ARN double brin lui permettra de reconnaître spécifiquement un fragment de l'ARNm cible du mécanisme de dégradation.
Les oligonucléotides d'ARN double brin pourront également présenter un squelette modifié lui conférant, par exemple, une meilleure stabilité.
Par exemple, les liaisons phosphodiester des brins d'ARN naturels peuvent être modifiées pour inclure au moins un atome d'azote ou de soufre. De plus, les oligonucléotides d'ARN double brin selon l'invention peuvent comprendre des bases autres que les 4 bases classiques.
La structure en double brin de l'oligonucléotide d'ARN double brin peut être obtenue par l'appariement de deux brins simples d'ARN complémentaires ou bien par un unique brin simple d'ARN "autocomplémentaire", c'est-à-dire comprenant deux fragments de séquences complémentaires pouvant s'apparier par repliement du brin simple pour former une double hélice.
A titre d'exemple d'oligonucléotides d'ARN double brin, on peut citer ceux décrits dans les demandes de brevet WO 00/44895, WO 01/36646, WO 99/32619, WO 01/29058, WO 00/44914 ou encore WO 03/101376
Il peut également s'agir d'oligonucléotides d'ARN double brin dit « stealth RNA » tels que ceux commercialisés par la société Invitrogen et décrits dans les demandes de brevet US2004014956 et US2004054155 qui sont des oligonucléotides d'ARN double brin pouvant être modifiés de telle sorte qu'une de ses extrémités, par exemple et de façon non limitative, comporte un groupement 2'-O-méthyl.
Les oligonucléotides d'ARN double brin peuvent être synthétisés selon de nombreuses méthodes de synthèse *in vivo* ou *in vitro* de façon manuelle ou automatique.

Les méthodes de synthèse *in vitro* peuvent être chimiques ou enzymatiques, par exemple en utilisant une ARN polymérase (à titre d'exemple de T3, T7 ou SP6) qui réalisera la transcription d'un modèle de séquence d'ADN (ou d'ADNc) choisi.

De nombreuses méthodes de synthèse *in vivo* d'ARN double brin sont décrites dans la littérature, elles peuvent être réalisées dans divers types cellulaires de bactéries ou d'organismes supérieurs (Sambrook et al. Molecular Cloning, A Laboratory Manual, Second Edition (1989), DNA cloning, volume I and II, D. N. Glover (ed. 1985), Oliginucleotide Synthesis, M. J. Gaits (ed. 1984), Nucleic Acid Hybridation, B. D. Hames and S. J. Higgins (ed.1984), Transcription and Translation B. D. Hames and S. J. Higgins (ed.1984), Animal Cell Culture, R. I. Freshney (ed. 1986), Immobilised Cells and Enzymes, IRL Press (1986), B. Pertal, A Practical Guide to Molecular Cloning (1984), Gene Transfer Vectors for Mammalian Cells, J. H. Miller and M. P. Calos, Cold Spring Harbor Laboratory (ed. 1987), Methods in Enzymology, vol. 154, Wu and Grossman, and 155, Wu, Mayer and Walker (1987), Immunochemical Methods in Cell and Molecular Biology, Academic Press, London, Scopes (1987), Protein Purification: Principle and Practice, 2nd ed., Springer-Verlag, N.-Y. and Handbook of Experimental Immunology, vol.I-IV, C. D. Weir and C. C. Blackwell (1986)). On pourra également se référer aux méthodes de synthèses décrites dans les demandes de brevet WO01/36646, WO01/75164 et US20030088087.

Par modèle de peau, on entend selon la présente invention des modèles de peaux reconstruites, par exemple, constituées d'un épiderme reconstruit à partir de kératinocytes de peau ou dérivés de la gaine externe du follicule pileux, d'un épiderme reconstruit sur un derme inerte dé-épidermisé (DED), d'un derme et d'un épiderme reconstruit, d'un épiderme reconstruit intégrant des cellules non kératinocytaires telles que les cellules de Langherans, les mélanocytes. A titre d'exemple, on peut citer le modèle de peau (épiderme) reconstruite Episkin®. Les modèles EpiSkin sont obtenus par culture de kératinocytes humains adultes sur un support collagénique dans des conditions permettant leur différenciation terminale et la reconstruction d'un épiderme avec une couche cornée fonctionnelle. Ces différents modèles in vitro sont notamment décrits dans les références suivantes : Asselineau D. et al., Exp Cell Res 1985 Aug ;159(2):536-539 ; Lenoir MC et al. Dev Biol 1988 Dec ;130 (2):610-20 ; Regnier M. et al. J Invest Dermatol 2000 Jan ;114 (1):220-220; Regnier M; et al. J Invest Dermatol 1997 Oct ;109 (4):510-2; Regnier M. et al. In Vitro Cell Dev Biol 1988 Jul ;24 (7):625-32; Cohen C. et al. J Invest Dermatol 1995 Jan ;104 (1):159-159; Tinois E. et al.,Exp Cell Res 1991 Apr ;193(2):310-319.

Les modèles de peau peuvent également être des peaux ex-vivo maintenues en survie tels que Episkin®, SkinEthic®, Matek®, Natskin®.

La présente description se rapporte à l'utilisation d'une composition comprenant au moins un oligonucléotide d'ARN double brin associé à au moins une particule cationique d'une taille inférieure ou égale à 1 µm, de potentiel zeta compris entre 10 et 80 mV choisie parmi les micelles de tensioactifs, les micelles de polymères blocs, les liposomes de tensioactifs non ionique et cationiques, les niosomes, les oléosomes, les particules de nanoémulsions, les nanocapsules, les particules organiques ou les particules inorganiques pour le traitement de modèle de peau, plus particulièrement pour inhiber partiellement ou totalement l'expression d'une protéine par les cellules d'un modèle de peau.

L'utilisation de ces modèles de peaux reconstruites permet ainsi, par une application topique ou systémique de siRNA vectorisés, de :
- valider une cible choisie en étudiant les effets produits par la diminution de l'expression de la protéine dans la peau ;
- reproduire des modèles de peaux pathologiques (exemple : ciblage des cytokératines CK5 ou CK14 pour reproduire l'Epidermolyse Bulleuse Simplex, CK1, CK2e ou CK10 pour les hyperkératoses épidermolytiques...)
- normaliser les modèles de peaux reconstruites en diminuant l'expression d'une protéine excédentaire par rapport à la peau normale (exemple : vimentine, CK17, ...)

A titre d'exemple non limitatif de protéine dont on pourra inhiber l'expression, on peut citer :
- les facteurs de croissance EGF, TNF-α, TGF, endothéline, NGF, HGF, IGF, VEGF ;
- les cytokines par exemple de type IL1, IL6, IL8 ... ;
- les récepteurs de type EGFr, TGFr, PAR, PPAR, FXR, RXR, CB1R, CB2R, VR1, CRAB2... ;
- les calcium binding proteins de type calmoduline, CLP, CLSP, celles de la famille des S100 proteins telles que S100A8, S100A9, S100A7 ... ;
- la calcineurine ;
- les transglutaminases par exemple les transglutaminases 1, 3 ou 5 ;
- les protéines assurant la cohésion/jonction intercellulaire telles occludines, laminines, caveolines, desmogleines, desmocollines, corneodesmosines, plakoglobines, desmoplakines... ;
- les enzymes impliquées dans les modifications postraductionnelles des protéines telles que les phosphatases ou protein-phosphatases par exemple la calcineurine, les phosphorylases, les proteins kinases (ex : PKC), les glucosyl transferases, les peptidyl-arginine-deiminase ... ;
- les protéases (MMP par exemple 1, 2, 3, 9, elastases, protéases à acide aspartique comme cathepsine-E, cathepsine-D, proteases à cystéine de type cathepsin-L, B ou H, cathepsine L2, SCCL, chymotrypsine équivalentes par exemple de type SCCE (Kallicrein 7), trypsin-like par exemple de type SCTE (Kallicrein 5), urokinase, SASPase, Caspase plus particulièrement caspase 14, calpaïnes, les protéases impliquées dans l'hydrolyse de la filaggrine de type subtilisin-like proprotein convertase comme la furin, PACE4, PC5/6 et PC7/8, les protéases de la famille des proteases à serine de type transmembranaire par exemple la matriptase et/ou leurs inhibiteurs endogènes comme TIMP, PAI1, PAI2, antileukoprotease, Elafin, LEKTI, cystatin A, cystatin M/E... ;
- les exo et les endoglycosidases par exemple de type heparanase, hyaluronidases, chondroîtinases, aspartyl glucosaminidase, B glycosidase, a glycosidases ... et leurs inhibiteurs endogènes ;
- les enzymes du métabolisme des lipides telles que HMGCoA reductase, cholestérol sulfatases ou sulfo transferases, sphingomyélinases, ceramidases... ;
- les enzymes impliqués dans la mélanogenèse telles que la tyrosinase, TRP-1 ou -2, SOX10, MITF, pMeI17, PAX3, POMC, MC1R, α-MSH, ...
- les enzymes impliqués dans le stress oxidatif telles que la SOD-1, SOD-2, glutathion peroxidase, catalase, thio-redoxine reductase, glutathion reductase, cytochromes P450, sulfotransferase, gama glutamyl synthetase, ...
- les enzymes du métabolisme des eicosanoides comme, par exemple, les cyclooxygénases, lipoxygénases, les phospholipases, 15-PGDH... ;
- les enzymes du métabolisme hormonal telle la 5 α-réductase de type 1 ou 2 ;
- les protéines matricielles de type élastines, collagène... ;
- les protéines de différenciation kératinocytaire de type cytokératine ;
- les protéines impliquées dans l'hydratation de la peau telle que la filaggrine, les aquaporines... ;
- les protéines impliquées dans les défenses antibactériennes de la peau hBD2, hBD3, dermcidin, RNase 7 ... ;
D'autres exemples non limitatifs, de protéines ou de peptides dont on vise à inhiber l'expression et/ou l'activité sont donnés dans « Textbook of dermatology » ed RH Champion JL Burton, DA Burns, SM Breathnach sixth edition 1998 Blackwell Science LtD ISBN 0-632-03796-2.

L'homme du métier choisira une concentration en oligonucléotide d'ARN double brin adapté à l'usage visé et à l'activité de l'oligonucléotide choisi. Sans que ce soit limitatif, la concentration en oligonucléotide d'ARN double brin pourra être comprise entre 10 pM et 1 µM.
Actuellement, pour des études sur cultures cellulaires en monocouche les moyens de transfection utilisés peuvent être soit la Lipofectanine 2000 (Invitrogen), JetSI (QBiogene), RNAiFect (Qiagen)... ou des particules virales type rétrovirus, lentivirus, adénovirus... Cependant, ces solutions de transfection sont toxiques, coûteuses et de disponibilité réduite, notamment car les particules virales nécessitent une mise en oeuvre très longue.
De plus, concernant la Lipofectamine 2000 ou les autres agents de transfection chimique, ils n'ont pas démontré, jusqu'à présent, qu'ils pouvaient permettre la pénétration de siRNA dans les cellules d'un modèle *in vitro* tridimensionnel telles que les peaux reconstruites (Episkin, Matek, Skinethic... ).
La demanderesse a pu montrer que les particules de la présente invention permettent de faire pénétrer des siRNA dans les cellules épidermiques d'un modèle de peau reconstruite (voir exemple 2). La présente invention consiste en un procédé de préparation d'un modèle de peau comprenant une étape d'administration dans le milieu de culture dudit modèle d'une composition comprenant au moins un oligonucléotide d'ARN double brin associé à au moins une particule cationique d'une taille inférieure ou égale à 1 µm, de potentiel zeta compris entre 10 et 80 mV choisie parmi les micelles de tensioactifs, les micelles de polymères blocs, les liposomes de tensioactifs non ionique et cationiques, les niosomes, les oléosomes, les particules de nanoémulsions, les nanocapsules, les particules organiques ou les particules inorganiques.
Le procédé pourra en outre comprendre une étape supplémentaire d'application d'un système occlusif ; une étape préalable visant à réduire le taux de Ca2+ dans le milieu de culture du modèle de peau ; un traitement pour que la perméabilité du modèle de peau soit augmentée en même temps que l'application de la composition.
Le plus souvent, la suspension de particules associées au siRNA est appliquée sur la surface du modèle avec un temps de contact d'au moins 2 h. Eventuellement, avant analyse, la surface peut être lavée.
Afin de faciliter la pénétration des particules associées au siRNA on pourra modifier la fonction du modèle en pré-traitement et/ou traitement par :
- utilisation d'un système occlusif comme par exemple une application de vaseline après application des particules associées au siRNA (une fois la surface sèche), ou couverture de la surface du modèle par une feuille de polymère occlusive. On veillera à ce que le moyen utilisé n'endommage pas la surface du modèle ;
- avant dépôt des particules associées au siRNA, on pourra réduire le taux de Ca2+ soit par le changement du milieu (0.10-0.75 mM au lieu de 1.5mM) soit par l'ajout d'un chélatant. Cela a pour effet de diminuer l'adhésion intermembranaire, augmentant ainsi la perméabilité du modèle (Effects of extra- and intracellular calcium concentration on DNA replication, lateral growth, and differentiation of human epidermal cells in culture. Virchows Arch B Cell Pathol Incl Mol Pathol. 1990;59(1):17-25*) ;*
- la iontophorèse, éléctroporation et toutes méthodes connues permettant d'augmenter la perméabilité du modèle.
L'application pourra se faire soit sur tous types cellulaires cutanés (kératinocytes, mélanocytes, fibroblastes...) en monocouche avant leur intégration ou non dans un modèle tridimensionnel, soit en topique sur le modèle tridimensionnel, soit en systémique dans le modèle tridimensionnel émergé par addition dans le milieu de culture. En variante, on pourra également immerger le modèle tridimensionnel dans un milieu contenant les particules associées au siRNA pour assurer la transfection.

Les figures suivantes illustrent les essais décrits dans l'exemple 2 :
**I. Ciblage cellulaire dans Episkin-J6**
   **Fig. I-1** **:** Block-It Fluorescent Oligo (DAPI)
   **Fig. I-2** **:** Block-It Fluorescent Oligo + Lipofectamine 2000 (DAPI)
   **Fig. I-3** **:** Block-It Fluorescent Oligo + E2 (x 40, DAPI)
   **Fig. I-4** **:** E2 (x 40, DAPI)
   **Fig. I-5** **:** Block-It Fluorescent Oligo + E2 (x 100, DAPI)
   **Fig. I-6** **:** Block-It Fluorescent Oligo + E2 (x 100)
**II. Ciblage cellulaire dans Episkin-J13**
   **Fig. II-1** : Block-It Fluorescent Oligo (x 40, DAPI)
   **Figures IIIA et IIIB** : visualisation de l'effet de formulation comprenant de l'octyle glucoside et du CTAB à différentes concentrations sur la viabilité de cellules HaCaT.

### EXEMPLE 1- Formulation

### Micelles cationigues:

A.1. Octyl β Glucoside / cetyl triammonium bromide au ratio molaire de 5/1, à partir de ce mélange, sont ensuite préparées quatre compositions aux taux de dilution ci-dessous :
   E1 : 100 mM dans l'eau distillée
   E2 : 50 mM dans l'eau distillée
   E3 : 25 mM dans l'eau distillée
   E4 : 12.5 mM dans l'eau distillée
      Les 2 tensioactifs (non ionique + cationique) sont solubilisés dans l'eau distillée. Une suspension de siRNA (20 µM) est ajoutée entre 1:1 et 1:9 volume (siRNA: micelles) réduisant ainsi la concentration micellaire en proportion.
A.2. Micelles de décyl β glucoside / behenyl triammonium chloride au ratio molaire de 5/1 dans l'eau distillée. Les mêmes concentrations que dans l'exemple précédent sont réalisées.

### Liposomes cationiques

- Exemple 1 : vésicules « fluides »

| | |
|---|---|
| Isostéarate de PEG 400 | 5.5% |
| Behenyl triammonium chloride | 0.5% |
| Eau distillée | qsp 100 |

- Exemple 2 : vésicules « rigides »

| | |
|---|---|
| Palmitate de sorbitan | 2.75% |
| Cholestérol | 2.75% |
| Behenyl triammonium chloride | 0.5% |
| Eau distillée | qsp 100 |

Ces deux exemples de suspensions de liposomes sont réalisés par dialyse. Les lipides constitutifs des vésicules sont solubilises dans une solution aqueuse d'octyl β glucoside. Cette solution est ensuite dialysée contre de l'eau pendant 72h.
La suspension de siRNA est ajoutée, ramenant la concentration en vésicule autour de 3% de lipide. Ceci n'étant qu'indicatif.

### Oléosomes cationiques

**Phase huile**

| | |
|---|---|
| Mono-di stéarate de sucrose commercialisé par Stéarainerie Dubois | 0.45% |
| Stéarate de sorbitane 4OE (Tween 61 Uniquema) | 0.30% |
| Béhényl triammonium chloride | 0.21% |
| Acétate de Vit E | 0.5% |
| Huile de jojoba | 0.5% |
| Stéaryl heptanoate | 1% |
| Huile se silicone volatile | 1% |
| Glycéride de Vit F | 0.5% |
| Conservateur | 0.02% |
| BHT | 0.01% |

**Phase aqueuse**

| | |
|---|---|
| Eau distillée | qsp 100 |
| Conservateur | 0.1% |

Cette dispersion est réalisée par homogénéisation haute pression afin d'avoir une taille de particule d'environ 170 nm. Est ajoutée ensuite, une suspension de siRNA (20µM) à des ratii allant de 1 :1 à 1 :20 (siRNA/Oléosomes). Le siRNA va alors se complexer à la surface des particules.

### Nanocapsules cationiques

**Phase organique**

| | |
|---|---|
| Polycaprolactone (MW :50000) | 1% |
| Vit E | 1% |
| Dimethicone copolyol DC2 5695 (Dow Corning) | 0.5% |
| Béhényl triammonium chloride | 0.21% |
| Acétone | 200 ml |

**Phase aqueuse**

| | |
|---|---|
| Pluronic F68 | 0.5% |
| Eau distillée | 200ml |

La phase organique est introduite sous agitation dans la phase aqueuse. L'acétone et 100 ml de phase aqueuse sont ensuite évaporés pour obtenir la suspension de nanocapsules dont la taille est de 220 nm. Est ajoutée ensuite, une suspension de siRNA (20µM) à des ratii allant de 1 :1 à 1 :20 (siRNA/nanocapsules). Le siRNA va alors se complexer à la surface des particules.

### Nanoparticules organiques

**Phase organique**

| | |
|---|---|
| Polyethylène adipate (Scientific Polymer products) | 2% |
| Dimethicone copolyol DC2 5695 (Dow Corning) | 0.5% |
| Béhényl triammonium chloride | 0.21% |
| Acétone | 200 ml |

**Phase aqueuse**

| | |
|---|---|
| Pluronic F68 | 0.5% |
| Eau distillée | 200ml |

La phase organique est introduite sous agitation dans la phase aqueuse. L'acétone et 100 ml de phase aqueuse sont ensuite évaporés pour obtenir la suspension de nanoparticules dont la taille est de 180 nm. Est ajoutée ensuite, une suspension de siRNA (20µM) à des ratii allant de 1 :1 à 1 :20 (siRNA/nanoparticules). Le siRNA va alors se complexer à la surface des particules.

### Nanoémulsion cationiques

**Phase Huile**

| | |
|---|---|
| Isostéarate de PEG 400 commercialisé par Uniqema | 1% |
| Béhényl triammonium chloride | 1% |
| Huile d'avocat | 1% |
| Huile de Jojaba | 3% |
| Cyclopentaméthylsiloxane | 2% |

| *Phase aqueuse* | |
|---|---|
| Eau distillée | 30% |
| Dipropylène glycol | 10% |

**Phase de dilution**

| | |
|---|---|
| Eau distillée | qsp 100% |
| Conservateur | 0.1% |

Une émulsion est réalisée en dispersant la phase huileuse sur la phase aqueuse sous très vive agitation. La suspension obtenue est ensuite homogénéisée plusieurs fois à l'aide d'un homogénéisateur très haute pression, à une pression d'environ 1200b. La taille des particules est de l'ordre de 50 nm et la suspension est transparente. La phase de dilution est ensuite ajoutée.
Comme dans les cas précédent, Est ajoutée ensuite, une suspension de siRNA (20µM) à des ratii allant de 1 :1 à 1 :20 (siRNA/nanoémulsion). Le siRNA va alors se complexer à la surface des particules.

### EXEMPLE 2 - CIBLAGE CELLULAIRE

Le ciblage cellulaire et tissulaire a été effectué dans le modèle d'épiderme reconstruit développé par EPISKIN SNC. Deux temps de cinétique de croissance du modèle ont été choisis pour l'application topique du complexe micelle/siRNA :
✔ 1 application à J6 de croissance de l'épiderme correspondant à un épiderme en début de stratification et de kératinisation.
✔ 1 application à J13 de croissance de l'épiderme correspondant à un épiderme stratifié et kératinisé.
Le siRNA choisi est le Block-It Fluorescent Oligo (20 µM), tel qu'il est décrit dans le manuel « Block-It Transfection Kit » (Réf cat N°: 13750-070, Invitrogen). Il s'agit d'un double brin d'ARN de 25 nucléotides couplé à la fluorescéine. La séquence codée n'a aucune homologie avec le génome humain et le rend donc non fonctionnel. Cet oligonucléotide a été spécifiquement élaboré pour l'étude du ciblage cellulaire. Lorsqu'il est transfecté, il est présent dans le cytoplasme et pénètre aussi dans le noyau cellulaire.

### Préparation du complexe micelle/siRNA :

3 µl de Block-It Fluorescent Oligo (20 µM), tel qu'il est décrit dans le manuel « Block-It Transfection Kit » (Réf cat N°: 13750-070, Invitrogen), sont mélangés à 9 µl de micelles cationiques E2 (Octyl β Glucoside / cetyl triammonium bromide au ratio molaire de 5/1, 50 mM dans l'eau distillée).

### Préparation du mélange Lipofectamine 2000/siRNA :

3 µl de Block-It Fluorescent Oligo (20 µM) sont dilués dans 5 µl d'OptiMEM (Invitrogen) et mélangés à 3 µl de Lipofectamine 2000 (Invitrogen) diluée dans 12 µl d'OptiMEM selon le protocole du fournisseur. La solution est incubée à température ambiante pendant 20 min pour permettre la complexation des liposomes et du siRNA.

### Préparation des solutions de contrôle :

✔ 3 µl de Block-It Fluorescent Oligo (20 µM) sont dilués dans 9 µl d'OptiMEM.
✔ 9 µl de micelles cationiques E2 (Octyl β Glucoside / cetyl triammonium bromide au ratio molaire de 5/1, 50 mM dans l'eau distillée) sont diluées dans 3 µl d'OptiMEM.

Les différents mélanges et solutions sont alors déposés sur différents échantillons d'épiderme EPISKIN et incubés en émersion 48 h à 37°C/5% CO2 dans du milieu de différenciation fourni avec le kit EPISKIN. Les échantillons sont alors congelés dans un mélange carboglace/Ethanol et coupés à l'aide d'un cryostat (MICROM HM560) en sections de 5 µm. Les coupes sont montés dans du milieu de montage Vectrashield (Vector Laboratories) contenant 1,5 µg de DAPI et visualisés en immunofluorescence avec les filtres appropriés pour la fluorescéine et le DAPI.

### Observations :

✔ Epiderme transfectés à J6 : on observe que, alors que la solution de Block-It Fluorescent Oligo (**Figure I-1**) ou le mélange Lipofectamine 2000 (**Figure I-2**) ne permet pas la pénétration de Block-It Fluorescent Oligo au-delà du *stratum corneum* en formation, le mélange E2/Block-It Fluorescent Oligo permet la pénétration du duplex RNA dans l'épiderme (**Figure I-3****,** **I-5, I-6**). Aucun marquage non spécifique n'est détecté lorsque E2 est appliqué seul (**Figure I-4**). Le caractère surprenant de cette observation est souligné par la présence homogène du Block-It Fluorescent Oligo dans toutes les cellules de l'épiderme alors qu'il est connu par l'homme de l'art que la transfection classique n'est efficace que dans les cellules en prolifération. Dans l'épiderme reconstruit, seules les cellules basales prolifèrent tandis que les cellules supra-basales se différencient.
✔ Epiderme transfectés à J13 : comme à J6, la solution de Block-It Fluorescent Oligo seule ne pénètre pas le *stratum corneum* (**Figure II-1**). Le mélange E2/ Block-It Fluorescent Oligo permet la pénétration du duplex fluorescent dans les différentes couches du *stratum comeum* et dans les différentes cellules de l'épiderme (**Figure II-2, II-3**) avec toutefois une efficacité et une homogénéité moindre qu'à J6.

### EXEMPLE 3 - EFFET D'UNE FORMULATION D'OCTYLE GLUCOSIDE ET DE CTAB A DIFFERENTES CONCENTRATIONS SUR LA MORTALITE DE CELLULES HaCaT

L'effet de la formule E2 : *50 mM micelles octyl glucoside* / *CTAB au ratio molaire* 5/1 est réalisée par incubation de 4 jours selon le protocole suivant sur cellules HaCaT (500 µl de milieu ensemencé à 40 000 cellules/puits, B-It : Block-It Fluorescent Oligo (Invitrogen).

40 000 cellules HaCaT/ puits sont ensemencées en duplicate dans une plaque 24 puits dans 500 µl de DMEM complet + 10% sérum de veau foetal. 1 µl de Block-It Fluorescent Oligo (Invitrogen) est dilué dans 1-15 µl d'une solution micellaire E2 composée de 50mM de micelles octyl glucoside / CTAB au ratio molaire de 5/1 et ajouté dans le milieu de culture soit une concentration finale de solution micellaire 0,1-1,5 mM.
Les cellules sont alors incubées 4 jours à 37°C avec un changement de milieu pour un échantillon des duplicates à J1 puis photographiées.

Les résultats obtenus sont représentés aux **figures IIIA et IIIB.**
Aucune différence n'est observée pour un même traitement entre les cellules avec ou sans changement de milieu à J1.
On observe une forte mortalité pour les concentrations de solution micellaire inférieure à la CMC (env. 1 mM) mettant en évidence l'effet toxique du véhicule sous sa forme libre tandis qu'une bonne viabilité cellulaire est observée au dessus de la CMC (1.5 mM).

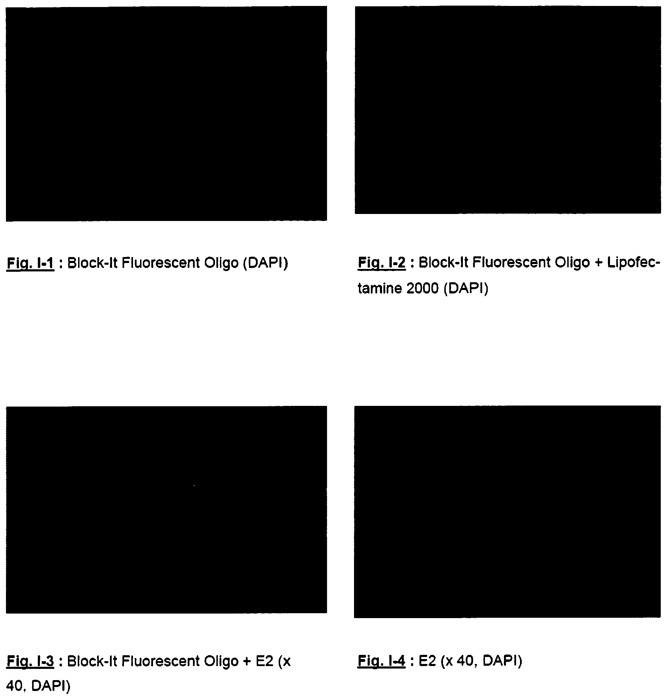

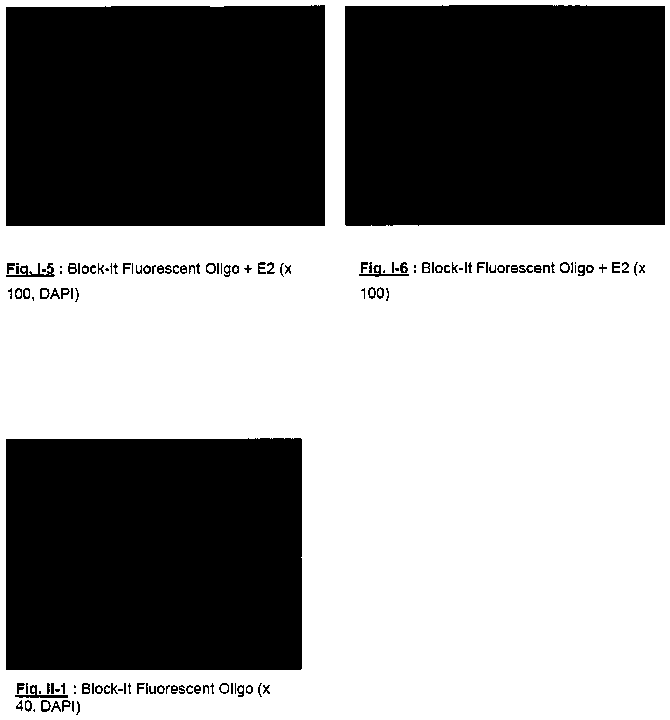

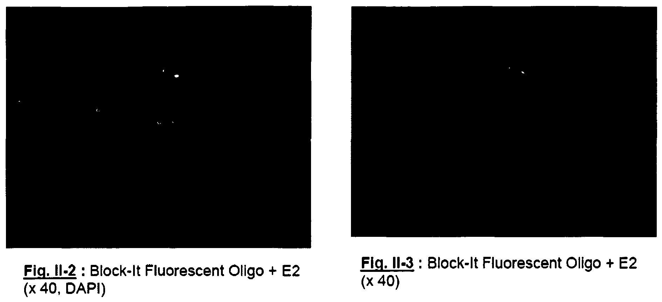

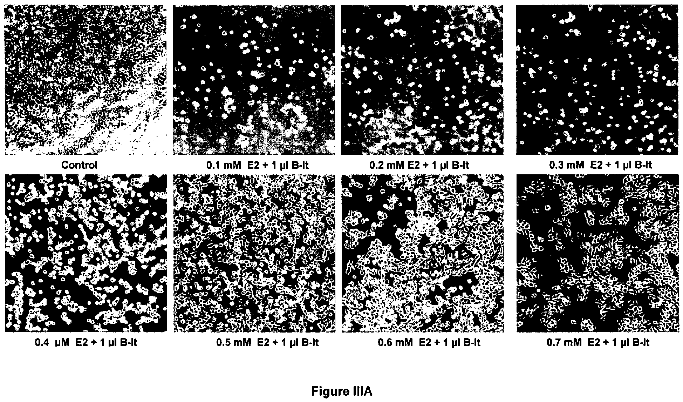

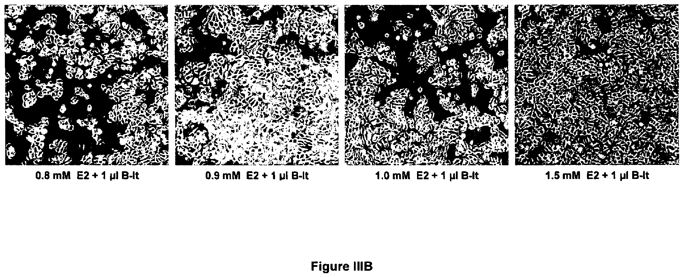

## Revendications

1. Procédé de préparation d'un modèle de peau, peaux reconstruites ou peaux ex-vivo maintenues en survie, comprenant une étape d'administration dans le milieu de culture dudit modèle d'une composition comprenant au moins un oligonucléotide d'ARN double brin associé à au moins une particule cationique d'une taille inférieure ou égale à 1 µm, de potentiel zeta compris entre 10 et 80 mV choisie parmi les micelles de tensioactifs, les micelles de polymères blocs, les liposomes de tensioactifs non ionique et cationiques, les niosomes, les oléosomes, les particules de nanoémulsions, les nanocapsules, les particules organiques ou les particules inorganiques.

2. Procédé selon la revendication 1, **caractérisé en ce qu'**il comprend l'étape supplémentaire d'application d'un système occlusif.

3. Procédé selon la revendication 1 ou 2, **caractérisé en ce qu'**il comprend l'étape préalable de réduire le taux de Ca2+ dans le milieu de culture du modèle de peau.

4. Procédé selon l'une quelconque des revendications 1 à 3, **caractérisé en ce que** la perméabilité du modèle dé peau est augmentée en même temps que l'application de ladite composition.

## Patentansprüche

1. Verfahren zur Herstellung eines Hautmodells, rekonstruierter Häute oder ex vivo am Überleben gehaltener Häute, bei dem man in einem Schritt in das Kulturmedium des Modells eine Zusammensetzung verabreicht, die mindestens ein doppelsträngiges DNA-Oligonukleotid in Verbindung mit mindestens einem kationischen Partikel mit einer Größe von weniger als oder gleich 1 µm, einem Zeta-Potenzial zwischen 10 und 80 mV, das aus Tensidmizellen, Blockpolymermizellen, Liposomen nicht-ionischer und kationischer Tenside, Niosomen, Oleosomen, Nanoemulsionspartikeln, Nanokapseln, organischen Partikeln oder anorganischen Partikeln ausgewählt ist, umfasst.

2. Verfahren nach Anspruch 1, **dadurch gekennzeichnet, dass** man in einem zusätzlichen Schritt ein okklusives System anwendet.

3. Verfahren nach Anspruch 1 oder 2, **dadurch gekennzeichnet, dass** man in einem vorangehenden Schritt den Anteil an Ca2+ im Kulturmedium des Hautmodells verringert.

4. Verfahren nach einem der Ansprüche 1 bis 3, **dadurch gekennzeichnet, dass** die Permeabilität des Hautmodells gleichzeitig mit der Anwendung der Zusammensetzung erhöht wird.

## Claims

1. Process for preparing a skin model, reconstructed skins or ex vivo skins maintained under survival conditions, comprising a step of administering in the culture medium of said model a composition comprising at least one double-stranded RNA oligonucleuotide associated with at least one cationic particle having a size of less than or equal to 1µm, and having a zeta potential of between 10 and 80 mV, chosen from surfactant micelles, block-polymer micelles, non-ionic and cationic surfactant liposomes, niosomes, oleosomes, nanoemulsion particles, nanocapsules, organic particles or inorganic particles.

2. Process according to Claim 1, **characterized in that** it comprises the additional step of applying an occlusive system.

3. Process according to Claim 1 or 2, **characterized in that** it comprises the preliminary step of reducing the Ca²⁺ level in the culture medium of the skin model.

4. Process according to any one of Claims 1 to 3, **characterized in that** the permeability of the skin model is increased at the same time as the application of said composition.
